# EUROPÄISCHE PATENTSCHRIFT

(11) **EP 4 031 526 B1**
(45) Veröffentlichungstag und Bekanntmachung des Hinweises auf die Patenterteilung: **10.07.2024**
(21) Anmeldenummer: 20768065.3
(22) Anmeldetag: 14.09.2020
(51) Int. Cl.: C07C 263/20, C07C 265/14

(54) **VERFAHREN ZUR HERSTELLUNG VON ISOCYANATEN**
METHOD FOR MAKING ISOCYANATES
PROCÉDÉ DE FABRICATION D'ISOCYANATES

(30) Priorität: 17.09.2019 EP 19197784; 05.02.2020 EP 20155540; 26.05.2020 EP 20176532
(43) Veröffentlichungstag der Anmeldung: 27.07.2022
(73) Patentinhaber: Covestro Deutschland AG, 51373 Leverkusen (DE)
(72) Erfinder: ZECHLIN, Joachim, 41472 Neuss (DE); LODDENKEMPER, Tim, 41542 Dormagen (DE); BLUDOWSKY, Thomas, 40885 Ratingen (DE)
(74) Vertreter: Levpat
(86) Internationale Anmeldenummer: PCT/EP2020/075591
(87) Internationale Veröffentlichungsnummer: WO 2021/052894

(56) Entgegenhaltungen:
- EP-A2- 1 717 223
- WO-A1-2019/145380

## Beschreibung

Die vorliegende Erfindung betrifft ein Verfahren zur Herstellung eines Isocyanats umfassend die Schritte **(A)** Umsetzen eines Amins mit einem stöchiometrischen Überschuss an Phosgen unter Einsatz eines aromatischen Lösungsmittels der Formel C₆H_{6-X}Cl_{X}, wobei X = 1 oder 2, (a) als Verdünnungsmittel während der Umsetzung und/oder (b) als Mittel zum Abkühlen des durch die Umsetzung des Amins mit Phosgen entstehenden Reaktionsgemisches (sog. *Quenche*), wobei (gegebenenfalls nach einer Entspannung) ein flüssiges Produktgemisch umfassend das Isocyanat und das eingesetzte aromatische Lösungsmittel sowie ein gasförmiges Produktgemisch enthaltend Phosgen und Chlorwasserstoff erhalten werden; gefolgt von **(B)** Isolieren des Isocyanats aus dem in Schritt (A) erhaltenen flüssigen Produktgemisch, umfassend den Schritt einer Rein-Destillation, in welcher das isolierte Isocyanat als Produktstrom anfällt, wobei in der Rein-Destillation oder in einem der Rein-Destillation vorgelagerten Destillationsschritt mindestens ein Strom umfassend aromatisches Lösungsmittel der Formel C₆H_{6-Y}Cl_{Y}, wobei Y = X + 1, (in Intervallen oder kontinuierlich) so ausgeschleust wird, dass das isolierte Isocyanat einen auf seine Gesamtmasse bezogenen Massenanteil an aromatischem Lösungsmittel der Formel C₆H_{6-Y}Cl_{Y} im Bereich von 0,0 ppm bis 9,9 ppm, bevorzugt 0,0 ppm bis 5,0 ppm, besonders bevorzugt 0,0 ppm bis 3,0 ppm aufweist.

Isocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen, wobei Phosgen im stöchiometrischen Überschuss eingesetzt wird. Die Umsetzung der Amine mit Phosgen kann sowohl in der Gasphase als auch in der Flüssigphase erfolgen, wobei die Reaktion diskontinuierlich oder kontinuierlich durchgeführt werden kann. Verfahren zur Herstellung von organischen Isocyanaten aus primären Aminen und Phosgen wurden bereits vielfach beschrieben. Von großtechnischem Interesse sind dabei sowohl aromatische Isocyanate, wie beispielsweise die Diamine der Diphenylmethanreihe (fortan MMDI - "monomeres MDI"), Gemische aus MMDI und den Polyaminen der Diphenylmethanreihe (das sind die höheren Homologen des MMDI, fortan PMDI, "polymeres MDI"; Gemische aus MMDI und PMDI werden fortan summarisch als MDI bezeichnet) oder Toluylendiisocyanat (TDI), als auch aliphatische oder cycloaliphatische Isocyanate wie z. B. 1,5-Pentandiisocyanat (PDI), 1,6-Hexamethylendiisocyanat (HDI) und Isophorondiisocyanat (IDPI). Daneben sind auch Isocyanate mit benzylischen Isocyanatgruppen (araliphatische Isocyanate) bedeutsam, insbesondere sei hier Xylylendiisocyanat (XDI) erwähnt.

Die moderne großtechnische Herstellung von Isocyanaten erfolgt semi-kontinuierlich (diskontinuierliche Durchführung eines Teils der Herstellungsschritte, z. B. diskontinuierliche Reaktion und kontinuierliche Aufarbeitung) oder kontinuierlich (alle Schritte kontinuierlich).

Die Verfahrensführung in der Flüssigphase, üblicherweise als Flüssigphasenphosgenierung bezeichnet, zeichnet sich dadurch aus, dass die Reaktionsbedingungen so gewählt werden, dass zumindest die Reaktionskomponenten Amin, Roh-Isocyanat und Phosgen, bevorzugt jedoch sämtliche Edukte, Produkte und Reaktionszwischenprodukte, bei den gewählten Bedingungen flüssig in einem geeigneten Lösungsmittel vorliegen. Nach erfolgter Umsetzung wird eine Gasphase enthaltend das Koppelprodukt Chlorwasserstoff und nicht umgesetztes (weil überstöchiometrisch eingesetztes) Phosgen abgetrennt; dabei verbleibt das angestrebte Isocyanat zusammen mit dem Lösungsmittel weitestgehend in der Flüssigphase. Das rohe Isocyanat fällt also im Gemisch mit Lösungsmittel als Flüssigkeitsstrom an, der zur Gewinnung von Rein-Isocyanat (und zur Rückgewinnung von Lösungsmittel sowie gelösten Anteilen von Phosgen und Chlorwasserstoff) aufgearbeitet wird.

Die Verfahrensführung in der Gasphase, üblicherweise als Gasphasenphosgenierung bezeichnet, zeichnet sich dadurch aus, dass die Reaktionsbedingungen so gewählt werden, dass zumindest die Reaktionskomponenten Amin, Isocyanat und Phosgen, bevorzugt jedoch sämtliche Edukte, Produkte und Reaktionszwischenprodukte, bei den gewählten Bedingungen gasförmig sind. Vorteile der Gasphasenphosgenierung sind u. a. ein verringertes Phosgenaufkommen (sog. Phosgen-"Hold-Up"), die Vermeidung schwer phosgenierbarer Zwischenprodukte, erhöhte Reaktionsausbeuten und ein geringerer Energiebedarf, da mit weniger Lösungsmittel gearbeitet wird. Das bei der Gasphasenphosgenierung zunächst gasförmig anfallende Reaktionsgemisch wird in einer sog. *Quenche* durch Inkontaktbringen mit einer Quenchflüssigkeit aus Lösungsmittel oder einem Isocyanat-Lösungsmittel-Gemisch so abgekühlt, dass das gewünschte Isocyanat größtenteils verflüssigt wird und eine Chlorwasserstoff- und Phosgen-haltige Gasphase verbleibt.

In allen großtechnisch relevanten Isocyanat-Herstellungsprozessen fällt demnach ein flüssiger RohIsocyanatstrom an, der zur Gewinnung des angestrebten Isocyanats in Reinform und zur Rückgewinnung anderer Wertstoffe wie Lösungsmittel aufgearbeitet werden muss. Diese Aufarbeitung umfasst im Allgemeinen die Abtrennung von Lösungsmittel, gelöstem Phosgen und gelöstem Chlorwasserstoff. Daran schließt sich eine Feinreinigung des Isocyanats an, die erforderlichenfalls auch eine Isomerentrennung umfassen kann. Abhängig von der Art des Isocyanats kann vor der Feinreinigung eine Homologentrennung durchgeführt werden. Hier ist insbesondere die teilweise Abtrennung von MMDI aus dem von Lösungsmittel, Phosgen und Chlorwasserstoff weitest gehend befreiten Isocyanat-Gemisch enthaltend MMDI und PMDI unter Erhalt einer MMDI-Fraktion, die PMDI allenfalls in unbedeutenden Spuren enthält (Roh-MMDI), und eines Gemisches aus PMDI und MMDI, zu nennen.

Die Aufarbeitung eines Rohisocyanatstroms im großtechnischen Maßstab ist nicht trivial, weil viele verschiedene Anforderungen gleichzeitig zu berücksichtigen sind. Neben der Gewinnung des Zielprodukts in möglichst reiner Form sind hier die möglichst verlustfreie Rückgewinnung von Phosgen, Chlorwasserstoff und Lösungsmittel zu nennen, insbesondere zum Zwecke der Rezyklierung derselben (ggf. nach weiterer Umsetzung wie etwa Chlorwasserstoff zu Chlor) in den Prozess. Dies alles muss unter möglichst wirtschaftlichen Bedingungen, d. h. unter möglichst geringem Energieverbrauch und möglichst geringem Verlust an Wertprodukt (insbesondere an Isocyanat, das bei nicht optimal ausgestalteter Aufarbeitung unerwünschte Folgereaktionen eingehen kann) erfolgen. Selbstredend muss dabei das angestrebte Isocyanat von Nebenprodukten, eingesetztem aromatischem Lösungsmittel, überschüssigem Phosgen und dergleichen so weit wie möglich befreit werden. Da sich als Lösungsmittel für die Isocyanatproduktion chlorierte aromatische Kohlenwasserstoffe (insbesondere Mono- oder Dichlorbenzol) bewährt haben, ist in diesem Zusammenhang zu erwähnen, dass die Anforderungen an Rein-Isocyanate bezüglich des Restgehalts an solchen chlorierten aromatischen Lösungsmitteln zunehmend strenger werden, womit auch die Herausforderungen an die Destillationstechnik steigen. Der Hintergrund für diese gestiegenen Anforderungen ist darin zu sehen, dass die aus den Isocyanaten produzierten Polyurethanprodukte keine gesundheitsschädlichen Ausdünstungen produzieren sollen, was insbesondere bei Schaumstoffen für Sitzpolster oder Matratzen von Bedeutung ist. Neben den eingesetzten aromatischen Lösungsmitteln selbst liegt dabei das Augenmerk im Stand der Technik auf deren vollständig chlorierten Reaktionsprodukten (im Falle des Einsatzes von Mono- oder Dichlorbenzol als Lösungsmittel also dem Hexachlorbenzol). Lösungsmittel mit im Vergleich zum eingesetzten aromatischen Lösungsmittel erhöhtem Chlorgehalt bilden sich durch Chlorierung des eingesetzten aromatischen Lösungsmittels in der Phosgenierung und/oder in der Aufarbeitung des rohen Isocyanats. Reaktionsprodukten des eingesetzten aromatischen Lösungsmittels, die nur einen zusätzlichen Chlorsubstituenten enthalten, wurden nach diesseitigem Kenntnisstand bisher im Stand der Technik keine besondere Aufmerksamkeit geschenkt.

Die Aufarbeitung von Rohisocyanaten wurde schon vielfach beschrieben:
Die internationale Patentanmeldung WO 2017/050776 A1 beschreibt die Aufarbeitung eines durch Flüssigphasenphosgenierung erhaltenen flüssigen Lösungsmittel-haltigen Roh-Isocyanat-Stroms durch Abtrennung von Phosgen und Chlorwasserstoff ("Entphosgenierung"), gefolgt von Abtrennung des Lösungsmittels, woran sich eine weitere Reinigung des rohen, von Phosgen, Chorwasserstoff und Lösungsmittel weitgehend befreiten Isocyanats durch Destillation anschließt. Im Fall des MDI umfasst diese Destillation auch eine Homologentrennung, in welcher MMDI unter Verbleib eines an MMDI abgereicherten PMDI/MMDI-Gemisches abgetrennt wird (sog. "Polymerabtrennung").

Möglichkeiten zur Aufarbeitung inklusive Homologen- und Isomerentrennung von MDI werden ausführlich in WO 2019/134909 A1 beschrieben. Möglichkeiten zur Aufarbeitung eines rohen TDI sind in WO 2018/114846 A1 sowohl für die Flüssig- als auch die Gasphasenphosgenierung beschrieben.

Die internationale Patentanmeldung WO 2019/145380 A1 beschreibt die Herstellung und Aufarbeitung aliphatischer, cycloaliphatischer und araliphatischer Isocyanate in der Gasphase gefolgt von einem raschen partiellen Verflüssigen ("Quenchen") des dabei anfallenden Produktgasgemisches mit einer Quenchflüssigkeit enthaltend ein aromatisches Lösungsmittel. Dabei fällt neben einem flüssigen Produktstrom umfassend das gewünschte Isocyanat ein Gasstrom umfassend Chlorwasserstoff und Phosgen an. Aus dem flüssigen Produktstrom wird das Isoycyanat durch mehrere Destillationsschritte isoliert. Überschüssiges Phosgen wird als Phosgengasstrom zurückgewonnen und in die Reaktion zurückgeführt. Die Schrift lehrt die Begrenzung des Gehalts an Benzol, Chlorbenzol und Dichlorbenzol in diesem zurückgewonnenen Phosgengasstrom auf einen Wert von 0,5 Gew.-% oder weniger. Hierdurch wird der Gehalt Hexachlorbenzol im herzustellenden Isocyanat reduziert (siehe die Beispiele der Anmeldung). Der Schrift liegt die Erkenntnis zugrunde, dass geringe Menge an Benzol, Chlorbenzol oder Dichlorbenzol in dem Gasstrom, der in die Reaktionszone eingeführt wird, zur Bildung *poly*chlorierter Aromaten führt, und zwar insbesondere zur Bildung des störenden Hexachlorbenzols. Mit *poly*chlorierten Aromaten sind offensichtlich die Reaktionsprodukte gemeint, die durch *Mehrfach*chlorierung von Benzol, Chlorbenzol und Dichlorbenzol gebildet werden, und zwar in der Reaktionszone gebildet werden (vgl. Seite 3, letzter Absatz).

Gemäß der Lehre dieser Schrift erfolgt das Zurückgewinnen überschüssigen Phosgens derart, dass zunächst das in der Reaktion anfallende Gasgemisch aus Chlorwasserstoff und Phosgen in einen gasförmigen Chlorwasserstoffstrom und einen flüssigen Phosgenstrom getrennt wird. Der flüssige Phosgenstrom wird im Anschluss partiell verdampft, was bevorzugt in einer Destillationskolonne durchgeführt wird. Diese Destillationskolonne kann am Kopf mit einer Aufgabestelle für flüssiges Frischphosgen versehen sein. Der Gehalt an Benzol, Chlorbenzol und Dichlorbenzol in dem gasförmigen Phosgenstrom, welcher der Destillationskolonne entnommen wird, kann durch eine geeignete Wahl der Austrittstemperatur dieses gasförmigen Phosgenstroms und gegebenenfalls durch geeignete Wahl der Menge an zusätzlich auf die Destillationskolonne aufgegebenem Frischphosgen auf den erfindungsgemäß vorgesehenen Wert von 0,5 Gew.-% oder weniger eingestellt werden. Weitere Maßnahmen zur Erreichung dieses Wertes nennt die Schrift nicht. Insbesondere lehrt die Schrift nicht, einen Strom an aromatischem Lösungsmittel, welches in der Reaktion *einfach* zusätzlich chloriert wurde, aus der Rein-Destillation des Isocyanats oder einem der Rein-Destillation vorgelagerten Destillationsschritt *aus dem gesamten Prozess heraus auszuschleusen.* WO 2019/145380 A1 gibt keinen Hinweis darauf, dass andere als vollständige Chlorierungsreaktionen überhaupt stattfinden, noch gibt diese Schrift einen Hinweis darauf, dass Chlorierungsreaktionen (welcher Art auch immer) *im Bereich der Aufarbeitung* eine Rolle spielen können (vgl. Seite 4, erster Absatz).

Die europäische Patentanmeldung EP 1 717 223 A2 beschreibt (vgl. Absatz [0015]) ein Verfahren zur Reinigung von Isocyanaten, bei dem man
a) einen Strom (1), enthaltend Isocyanat, höher und niedrigersiedende Komponenten, sowie nicht verdampfbaren Rückstand, in einer mindestens eine theoretische Trennstufe enthaltenden Destillation in einen Teilstrom (2), der einen nicht verdampfbaren Rückstand und Isocyanat enthält, und in einen Isocyanat und Leichtsieder enthaltenden Brüdenstrom (3) auftrennt,
b) den nicht verdampfbaren Rückstand im Teilstrom (2) von dem Brüdenstrom (3) und/oder von Stoffströmen, die den Brüdenstrom (3) zumindest teilweise enthalten, getrennt hält,
c) aus dem Teilstrom (2) mindestens einen weiteren isocyanathaltigen Brüdenstrom (4) und einen im wesentlichen nicht verdampfbaren Rückstand enthaltenden Strom (8) auftrennt und
d) den oder die isocyanathaltigen Brüdenströme (4) und den Brüdenstrom (3) aus a) in drei Einzelströme (5, 6, 7) mit unterschiedlichen Siedebereichen destillativ auftrennt, wobei der am leichtesten siedende Strom (5) einen wesentlichen Teil des Leichtsiederanteils des Rohisocyanatstromes (1) enthält, der am schwersten siedende Strom (7) einen wesentlichen Teil des Hochsiederanteils des Rohisocyanatstromes (1) enthält, und der mittelsiedende Strom (6) im wesentlichen Wertprodukt enthält.

Dabei ist der Strom (1), der zu reinigende Rohisocyanatstrom, im Fall der Herstellung des Isocyanats durch Phosgenierung, was bevorzugt ist, ein Strom, aus welchem Chlorwasserstoff, Phosgen und Lösungsmittel im Wesentlichen abgetrennt sind, sodass der Gehalt an Chlorwasserstoff und Phosgen im Strom (1) jeweils unter 1000 ppm liegt und der Lösungsmittelgehalt unter 1 Gew.-%, bevorzugt unter 0,5 Gew.-% und besonders bevorzugt unter 0,1 Gew.-% liegt. Der Rohisocyanatstrom (1) enthält neben dem als Wertprodukt zu gewinnenden Isocyanat üblicherweise 100 ppm bis 5 % leichter als das Isocyanat siedende Komponenten (Leichtsieder), 100 bis 5000 ppm höher als das Isocyanat siedende Komponenten (Hochsieder), deren Siedepunkt bei Normaldruck jedoch nicht mehr als 60 °C höher ist als der Siedepunkt des Isocyanates, und 1 bis 8 Gew.-% nicht verdampfbaren Rückstand polymerer Natur, d. h., das Produkt pyrolisiert bevor es bei Normaldruck verdampft. Das - im Vergleich zum Isocyanat leicht siedende - Lösungsmittel ist also im Rohisocyanatstrom (1) allenfalls noch in Spuren vorhanden, sodass mit Leichtsiedern in diesem Zusammenhang offenbar *andere Komponenten* gemeint sind. Konkret genannt werden u. a. chlorhaltige Nebenprodukte, die durch Weiterreaktion *des Isocyanats* (und nicht des Lösungsmittels) entstehen (beispielsweise die beiden chlorhaltigen Nebenkomponenten 1-lsocyanato-6-Chlor-Hexan oder 1,6-Dichlorhexan im Falle der Herstellung von 1,6-Diisocyanatohexan). Die Chlorierung *von Lösungsmittel* wird in der Schrift nicht erwähnt, weder eine partielle noch eine vollständige Chlorierung. Als typische Leichtsieder im Fall von aromatischen Isocyanaten werden Methylphenylisocyanat (Herstellung von TDI) und Phenylisocyanat (Herstellung von MDI) genannt.

In einer bevorzugten Ausgestaltung des beschriebenen Verfahrens, die in FIG. 2 dargestellt ist, wird der Rohisocyanatstrom (1) seitlich in eine Trennwandkolonne geführt und in dieser in die drei Ströme 5, 6 und 7 aufgetrennt, wobei der am leichtesten siedende Strom (5) oberhalb der Trennwand am Kopf der Kolonne, der mittelsiedende Strom (6) (= der Wertproduktstrom) im Bereich der Trennwand oberhalb des Zulaufs und der am schwersten siedende Strom (7) im Sumpf der Kolonne entnommen werden. Im Beispiel werden typische Zusammensetzungen dieser Ströme für den Fall der Herstellung von TDI genannt:
Der am leichtesten siedende Strom (5) enthält (neben beträchtlichen Anteile an Isocyanat, nämlich 99,4 Gew.-% TDI) 0,5 Gew.-% Leichtsieder; woraus die verbleibenden 0,1 Gew.-% bestehen, wird nicht angegeben (siehe die obere Tabelle auf Seite 7). Der mittelsiedende Strom (6) enthält 99,9 Gew.-TDI, 10 ppm Leichtsieder und 50 ppm höhersiedende Komponenten. Woraus die verbleibenden 0,094 Gew.-% (940 ppm) des mittelsiedenden Stroms (6) bestehen, wird nicht angegeben. Der am schwersten siedende Strom (7) besteht zu 30 Gew.-% aus TDI und zu 70 Gew.-% aus höhersiedenden Komponenten.

Es hat sich nun herausgestellt, dass im Zusammenhang mit der Zielsetzung der Gewinnung von Isocyanaten mit möglichst geringem Restanteil an Lösungsmittel zum Zwecke der Vermeidung gesundheitsschädlicher Ausdünstungen in Polyurethanfolgeprodukten weitere Faktoren eine Rolle spielen. Es bestand daher ein Bedarf an weiteren Verbesserungen auf diesem Gebiet.

Dem geschilderten Bedarf Rechnung tragend ist daher ein Gegenstand der vorliegenden Erfindung ein Verfahren zur Herstellung eines Isocyanats umfassend die Schritte:
(A) Umsetzen eines (aromatischen, aliphatischen, cycloaliphatischen oder araliphatischen, bevorzugt aromatischen) Amins mit einem stöchiometrischen Überschuss an Phosgen unter Einsatz eines aromatischen Lösungsmittels der Formel C₆H_{6-X}Cl_{X}, wobei X = 1 oder 2,
   (a) als Verdünnungsmittel während der Umsetzung und/oder
   (b) als Mittel zum Abkühlen des durch die Umsetzung des Amins mit Phosgen entstehenden Reaktionsgemisches (sog. *Quenche*),
   wobei (gegebenenfalls nach einer Entspannung) ein flüssiges Produktgemisch umfassend das Isocyanat und das eingesetzte aromatische Lösungsmittel sowie ein gasförmiges Produktgemisch enthaltend Phosgen und Chlorwasserstoff erhalten werden;
(B) Isolieren des Isocyanats aus dem in Schritt (A) erhaltenen flüssigen Produktgemisch, umfassend den Schritt einer Rein-Destillation, in welcher das isolierte Isocyanat als Produktstrom anfällt, wobei in der Rein-Destillation oder in einem der Rein-Destillation vorgelagerten Destillationsschritt mindestens ein Strom umfassend aromatisches Lösungsmittel der Formel C₆H_{6-Y}Cl_{Y}, wobei Y = X + 1, (in Intervallen oder kontinuierlich) so ausgeschleust (d. h. aus dem Verfahren zur Herstellung des Isocyanats *heraus* geführt) wird, dass das isolierte Isocyanat einen auf seine Gesamtmasse bezogenen Massenanteil an aromatischem Lösungsmittel der Formel C₆H_{6-Y}Cl_{Y} im Bereich von 0,0 ppm bis 9,9 ppm, bevorzugt 0,0 ppm bis 5,0 ppm, besonders bevorzugt 0,0 ppm bis 3,0 ppm aufweist.

Es wurde nämlich gefunden, dass neben dem eingesetzten aromatischen Lösungsmittel (C₆H_{6-X}Cl_{X}) auch dessen Folgeprodukte mit nur einem zusätzlichen Chlorsubstituenten (einfach zusätzlich chloriertes aromatisches Lösungsmittel C₆H_{6-Y}Cl_{Y}) ausreichend von Isocyanat-Zielprodukt abgetrennt werden müssen, was aufgrund der im Stand der Technik üblichen vielfachen Lösungsmittel-Rückführungen innerhalb eines Isocyanat-Herstellprozesses eine Abführung dieses einfach zusätzlich chlorierten aromatischen Lösungsmittels C₆H_{6-Y}Cl_{Y} *aus dem Prozess heraus* (= Ausschleusung) erfordert. Bei der Bereitstellung von Isocyanaten mit ausreichender Reinheit spielen nämlich auch diese einfach aufchlorierten Lösemittel C₆H_{6-Y}Cl_{Y} eine entscheidende Rolle, um Polyurethanprodukte ohne gesundheitsbedenkliche Ausdünstungen gewährleisten zu können. Wird neben einer Begrenzung des Gehalts an eingesetztem aromatischem Lösungsmittel (C₆H_{6-X}Cl_{X}) im herzustellenden Isocyanat, die im Stand der Technik üblich ist, auch der Gehalt an einfach zusätzlich chloriertem aromatischem Lösungsmittel (C₆H_{6-Y}Cl_{Y}) begrenzt, und zwar auf 9,9 ppm oder weniger, wie erfindungsgemäß vorgesehen, ist davon auszugehen, dass der Gesamtgehalt an Chloraromaten (d. h. das eingesetzte Lösungsmittel und dessen sämtliche Chlorierungsprodukte) ausreichend gering ist.

Der auf *die Gesamtmasse bezogene Massenanteil an aromatischem Lösungsmittel der Formel C₆H_{6-Y}Cl_{Y} im isolierten Isocyanat* kann grundsätzlich nach allen dem Fachmann für die Konzentrationsbestimmung niedermolekularer organischer Verbindungen geläufigen Methoden ermittelt werden. Diese liefern im Allgemeinen im Rahmen der für die Zwecke der vorliegenden Erfindung erforderlichen Genauigkeit übereinstimmende Resultate. Bevorzugt ist die Messung durch Gaschromatographie unter Einsatz eines Flammenionisationsdetektors (FID-Detektor) oder Elektroneneinfangdetektors (ECD-Detektor). Im Zweifelsfall ist der durch Gaschromatographie unter Einsatz eines Flammenionisationsdetektors bestimmte Wert maßgeblich.

Der Begriff *"Ausschleusung"* und davon abgeleitete Verben und Adjektive beziehen sich im Rahmen der vorliegenden Erfindung auf die Abführung eines im Prozess (d. h. im Verfahren zur Herstellung eines Isocyanats) anfallenden Stromes *aus dem Prozess* (d. h. aus dem Verfahren zur Herstellung des Isocyanats) *heraus*; d. h. derart ausgeschleuste Ströme werden nicht wieder in das Verfahren zurückgeführt, weder in den Reaktions- noch in den Aufarbeitungsteil. Ausgeschleuste Ströme werden bevorzugt entsorgt, insbesondere verbrannt. Eine stoffliche Verwendung derartiger ausgeschleuster Ströme ist jedoch, mit Ausnahme ihrer Rückführung in das Verfahren zur Herstellung des Isocyanats, nicht ausgeschlossen. Ausgeschleuste Ströme enthalten aromatisches Lösungsmittel der Formel C₆H_{6-Y}Cl_{Y} insbesondere in einem auf ihre Gesamtmasse bezogenen Massenanteil im Bereich von 1,0 % bis 10 %, bevorzugt im Bereich von 1,5 % bis 5,0 %, besonders bevorzugt im Bereich von 2,2 % bis 3,0 %.

Es folgt zunächst eine Kurzzusammenfassung verschiedener möglicher Ausführungsformen der Erfindung.

In einer **ersten Ausführungsform** der Erfindung, die mit allen anderen Ausführungsformen kombiniert werden kann, durchläuft das in Schritt (A) erhaltene flüssige Produktgemisch in Schritt (B) vor der Rein-Destillation eine Entphosgenierung zur Abtrennung gelösten Phosgens.

In einer **zweiten Ausführungsform** der Erfindung, die mit allen anderen Ausführungsformen kombiniert werden kann, wird das in Schritt (A) erhaltene gasförmige Produktgemisch in Schritt (B) einer Wäsche mit aromatischem Lösungsmittel der Formel C₆H_{6-X}Cl_{X} zur Abtrennung von Isocyanat unterzogen.

In einer **dritten Ausführungsform** der Erfindung, die mit allen anderen Ausführungsformen kombiniert werden kann, durchläuft das in Schritt (A) erhaltene flüssige Produktgemisch in Schritt (B) vor der Rein-Destillation eine Lösungsmittel-Destillation zur Abtrennung von aromatischem Lösungsmittel der Formel C₆H_{6-X}Cl_{X} (wobei die Lösungsmittel-Destillation eine weitere Destillation zur Reinigung des abgetrennten aromatischen Lösungsmittels der Formel C₆H_{6-X}Cl_{X} umfassen kann).

In einer **vierten Ausführungsform** der Erfindung, die eine besondere Ausgestaltung der dritten Ausführungsform ist, wird das in der Lösungsmittel-Destillation abgetrennte aromatische Lösungsmittel der Formel C₆H_{6-X}Cl_{X} einer Lösungsmittel-Reinigung zur Abtrennung von darin enthaltenem Phosgen (Lösungsmittel-Entphosgenierung) unterzogen.

In einer **fünften Ausführungsform** der Erfindung, die mit allen anderen Ausführungsformen kombiniert werden kann, sofern diese nicht den Einsatz einer Trennwandkolonne in der Rein-Destillation ausschließen, wird in Schritt (B) die Rein-Destillation in einer Trennwandkolonne durchgeführt, wobei in einem Seitenabzug der Trennwandkolonne der Produktstrom an Isocyanat und am Kopf der Trennwandkolonne aromatisches Lösungsmittel der Formel C₆H_{6-X}Cl_{X} erhalten werden (wobei die Rein-Destillation eine weitere Destillationskolonne zur Reinigung des am Kopf der Trennwandkolonne erhaltenen aromatischen Lösungsmittels der Formel C₆H_{6-X}Cl_{X} umfassen kann).

In einer **sechsten Ausführungsform** der Erfindung, die mit allen anderen Ausführungsformen kombiniert werden kann, sofern diese nicht den Einsatz einer Trennwandkolonne in der Rein-Destillation vorsehen, wird in Schritt (B) die Rein-Destillation in zwei hintereinandergeschalteten Destillationskolonnen ohne Trennwand durchgeführt, wobei am Kopf der ersten Destillationskolonne aromatisches Lösungsmittel der Formel C₆H_{6-X}Cl_{X} und als Destillat der zweiten Destillationskolonne der Produktstrom an Isocyanat erhalten werden (wobei die Rein-Destillation eine weitere Destillationskolonne zur Reinigung des am Kopf der ersten Destillationskolonne erhaltenen aromatischen Lösungsmittels der Formel C₆H_{6-X}Cl_{X} umfassen kann).

In einer **siebten Ausführungsform** der Erfindung, die mit allen anderen Ausführungsformen kombiniert werden kann, sofern diese eine Lösungsmittel-Destillation umfassen, wird das in der Lösungsmittel-Destillation abgetrennte aromatische Lösungsmittel der Formel C₆H_{6-X}Cl_{X} in einem Gemisch mit aromatischem Lösungsmittel der Formel C₆H_{6-Y}Cl_{Y} erhalten (= *das in der Lösungsmittel-Destillation erhaltene Gemisch*), wobei ein erster Teil dieses Gemisches (optional nach Lösungsmittel-Entphosgenierung) in Schritt (A) zurückgeführt und ein zweiter Teil dieses Gemisches nicht in Schritt (A) zurückgeführt, sondern (in Intervallen oder kontinuierlich) ausgeschleust wird.

In einer **achten Ausführungsform** der Erfindung, die eine Alternative zur siebten und nachfolgend geschilderten neuten Ausführungsform ist, ansonsten jedoch mit allen anderen Ausführungsformen kombiniert werden kann, sofern diese eine Lösungsmittel-Destillation umfassen, wird das in der Lösungsmittel-Destillation abgetrennte aromatische Lösungsmittel der Formel C₆H_{6-X}Cl_{X} in einem Gemisch mit aromatischem Lösungsmittel der Formel C₆H_{6-Y}Cl_{Y} erhalten (= *das in der Lösungsmittel-Destillation erhaltene Gemisch*), wobei ein erster Teil dieses Gemisches (optional nach Lösungsmittel-Entphosgenierung) in Schritt (A) zurückgeführt und ein zweiter Teil dieses Gemisches in einer weiteren Destillation gereinigt wird, wobei aromatisches Lösungsmittel der Formel C₆H_{6-X}Cl_{X} aus dem zweiten Teil dieses Gemisches abgetrennt und anschließend (zumindest teilweise, insbesondere vollständig) in Schritt (A) zurückgeführt wird, wobei der nach der Abtrennung des aromatischen Lösungsmittels der Formel C₆H_{6-X}Cl_{X} aus dem zweiten Teil dieses Gemisches verbleibende (an aromatischem Lösungsmittel der Formel C₆H_{6-Y}Cl_{Y} angereicherte) Teil des zweiten Teils dieses Gemisches (in Intervallen oder kontinuierlich) ausgeschleust wird.

In einer **neunten Ausführungsform** der Erfindung, die eine Alternative zur siebten und achten Ausführungsform ist, ansonsten jedoch mit allen anderen Ausführungsformen kombiniert werden kann, sofern diese eine Lösungsmittel-Destillation umfassen, wird das in der Lösungsmittel-Destillation abgetrennte aromatische Lösungsmittel der Formel C₆H_{6-X}Cl_{X} in einem Gemisch mit aromatischem Lösungsmittel der Formel C₆H_{6-Y}Cl_{Y} erhalten (= *das in der Lösungsmittel-Destillation erhaltene Gemisch*), wobei dieses Gemisch in einer weiteren Destillation gereinigt wird, wobei aromatisches Lösungsmittel der Formel C₆H_{6-X}Cl_{X} aus diesem Gemisch abgetrennt und anschließend (zumindest teilweise, insbesondere vollständig) in Schritt (A) zurückgeführt wird, wobei der nach der Abtrennung des aromatischen Lösungsmittels der Formel C₆H_{6-X}Cl_{X} aus diesem Gemisch verbleibende (an aromatischem Lösungsmittel der Formel C₆H_{6-Y}Cl_{Y} angereicherte) Teil dieses Gemisches (in Intervallen oder kontinuierlich) ausgeschleust wird.

In einer **zehnten Ausführungsform** der Erfindung, die eine besondere Ausgestaltung der fünften bzw. sechsten Ausführungsform ist, fällt das am Kopf der Trennwandkolonne oder am Kopf der ersten Destillationskolonne erhaltene aromatische Lösungsmittel der Formel C₆H_{6-X}Cl_{X} in einem Gemisch mit aromatischem Lösungsmittel der Formel C₆H_{6-Y}Cl_{Y} an (= *das am Kopf der Trennwandkolonne oder am Kopf der ersten Destillationskolonne erhaltene Gemisch).*

In einer **elften Ausführungsform** der Erfindung, die eine erste besondere Ausgestaltung der zehnten Ausführungsform ist, wird das anfallende Gemisch (d. h. das am Kopf der Trennwandkolonne oder am Kopf der ersten Destillationskolonne erhaltene Gemisch) ausgeschleust.

In einer **zwölften Ausführungsform** der Erfindung, die eine zweite besondere Ausgestaltung der zehnten Ausführungsform ist, wird ein erster Teil des am Kopf der Trennwandkolonne oder am Kopf der ersten Destillationskolonne erhaltenen Gemisches in Schritt (A) zurückgeführt, und ein zweiter Teil dieses Gemisches wird nicht in Schritt (A) zurückgeführt, sondern (in Intervallen oder kontinuierlich) ausgeschleust.

In einer **dreizehnten Ausführungsform** der Erfindung, die eine dritte besondere Ausgestaltung der zehnten Ausführungsform ist, und zwar für den Fall, dass Schritt (B) einen Schritt der Wäsche des in Schritt (A) erhaltenen gasförmigen Produktgemisches mit aromatischem Lösungsmittel der Formel C₆H_{6-X}Cl_{X} zur Abtrennung von Isocyanat (= zweite oben geschilderte Ausführungsform) umfasst, wird ein erster Teil des am Kopf der Trennwandkolonne oder am Kopf der ersten Destillationskolonne erhaltenen Gemisches in den Schritt der Wäsche zurückgeführt, und ein zweiter Teil dieses Gemisches wird nicht in den Schritt der Wäsche zurückgeführt, sondern (in Intervallen oder kontinuierlich) ausgeschleust.

In einer **vierzehnten Ausführungsform** der Erfindung, die eine vierte besondere Ausgestaltung der zehnten Ausführungsform ist, wird ein erster Teil des am Kopf der Trennwandkolonne oder am Kopf der ersten Destillationskolonne erhaltenen Gemisches in Schritt (A) zurückgeführt, und ein zweiter Teil dieses Gemisches wird in einer weiteren Destillation gereinigt, wobei aromatisches Lösungsmittel der Formel C₆H_{6-X}Cl_{X} aus dem zweiten Teil dieses Gemisches abgetrennt und anschließend (zumindest teilweise, insbesondere vollständig) in Schritt (A) zurückgeführt wird, wobei der nach der Abtrennung des aromatischen Lösungsmittels der Formel C₆H_{6-X}Cl_{X} aus dem zweiten Teil des Gemisches verbleibende (an aromatischem Lösungsmittel der Formel C₆H_{6-Y}Cl_{Y} angereicherte) Teil des zweiten Teils dieses Gemisches (in Intervallen oder kontinuierlich) ausgeschleust wird.

In einer **fünfzehnten Ausführungsform** der Erfindung, die eine fünfte besondere Ausgestaltung der zehnten Ausführungsform ist, und zwar für den Fall, dass Schritt (B) einen Schritt der Wäsche des in Schritt (A) erhaltenen gasförmigen Produktgemisches mit aromatischem Lösungsmittel der Formel C₆H_{6-X}Cl_{X} zur Abtrennung von Isocyanat (= zweite oben geschilderte Ausführungsform) umfasst, wird ein erster Teil des am Kopf der Trennwandkolonne oder am Kopf der ersten Destillationskolonne erhaltenen Gemisches in den Schritt der Wäsche zurückgeführt, und ein zweiter Teil dieses Gemisches wird in einer weiteren Destillation gereinigt, wobei aromatisches Lösungsmittel der Formel C₆H_{6-X}Cl_{X} aus dem zweiten Teil dieses Gemisches abgetrennt und anschließend (zumindest teilweise, insbesondere vollständig) in den Schritt der Wäsche oder in Schritt (A) zurückgeführt wird, wobei der nach der Abtrennung des aromatischen Lösungsmittels der Formel C₆H_{6-X}Cl_{X} aus dem zweiten Teil dieses Gemisches verbleibende (an aromatischem Lösungsmittel der Formel C₆H_{6-Y}Cl_{Y} angereicherte) Teil des zweiten Teils dieses Gemisches (in Intervallen oder kontinuierlich) ausgeschleust wird.

In einer **sechzehnten Ausführungsform** der Erfindung, die eine sechste besondere Ausgestaltung der zehnten Ausführungsform ist, wird das am Kopf der Trennwandkolonne oder am Kopf der ersten Destillationskolonne erhaltene Gemisch in einer weiteren Destillation gereinigt, wobei aromatisches Lösungsmittel der Formel C₆H_{6-X}Cl_{X} aus diesem Gemisch abgetrennt und anschließend (zumindest teilweise, insbesondere vollständig) in Schritt (A) zurückgeführt wird, wobei der nach der Abtrennung des aromatischen Lösungsmittels der Formel C₆H_{6-X}Cl_{X} aus diesem Gemisch verbleibende (an aromatischem Lösungsmittel der Formel C₆H_{6-Y}Cl_{Y} angereicherte) Teil dieses Gemisches (in Intervallen oder kontinuierlich) ausgeschleust wird.

In einer **siebzehnten Ausführungsform** der Erfindung, die eine siebte besondere Ausgestaltung der zehnten Ausführungsform ist, und zwar für den Fall, dass Schritt (B) einen Schritt der Wäsche des in Schritt (A) erhaltenen gasförmigen Produktgemisches mit aromatischem Lösungsmittel der Formel C₆H_{6-X}Cl_{X} zur Abtrennung von Isocyanat (= zweite oben geschilderte Ausführungsform) umfasst, wird das am Kopf der Trennwandkolonne oder am Kopf der ersten Destillationskolonne erhaltene Gemisch in einer weiteren Destillation gereinigt, wobei aromatisches Lösungsmittel der Formel C₆H_{6-X}Cl_{X} aus diesem Gemisch abgetrennt und (zumindest teilweise, insbesondere vollständig) in den Schritt der Wäsche oder in Schritt (A) zurückgeführt wird, wobei der nach der Abtrennung des aromatischen Lösungsmittels der Formel C₆H_{6-X}Cl_{X} aus diesem Gemisch verbleibende (an aromatischem Lösungsmittel der Formel C₆H_{6-Y}Cl_{Y} angereicherte) Teil dieses Gemisches (in Intervallen oder kontinuierlich) ausgeschleust wird.

In einer **achtzehnten Ausführungsform** der Erfindung, die mit allen anderen Ausführungsformen kombiniert werden kann, sofern diese eine Lösungsmittel-Destillation umfassen, in welcher das in der Lösungsmittel-Destillation abgetrennte aromatische Lösungsmittel der Formel C₆H_{6-X}Cl_{X} in einem Gemisch mit aromatischem Lösungsmittel der Formel C₆H_{6-Y}Cl_{Y} erhalten wird (= *das in der Lösungsmittel-Destillation erhaltene Gemisch*), wobei dieses Gemisch teilweise oder vollständig ausgeschleust wird, wird der ausgeschleuste Teil dieses Gemisches bzw. das ausgeschleuste Gemisch verbrannt.

In einer **neunzehnten Ausführungsform** der Erfindung, die mit allen anderen Ausführungsformen kombiniert werden kann, sind Rohrleitungen, die zur Verbindung eines zur Aufnahme des flüssigen Produktgemisches aus Schritt (A) eingesetzten Tankbehälters mit zur Durchführung von Schritt (B) eingesetzten Destillationseinrichtungen und/oder zur Verbindung dieser Destillationseinrichtungen untereinander eingesetzt werden, aus Edelstahl des Typs 2.4610, 1.4529 oder 1.4539 gefertigt.

In einer **zwanzigsten Ausführungsform** der Erfindung, die eine besondere Ausgestaltung der neunzehnten Ausführungsform ist, wird der Schritt der Rein-Destillation, in dem der Produktstrom an isoliertem Isocyanat erhalten wird, in einer Destillationskolonne durchgeführt, die einen Behälter zur Aufnahme flüssigen Destillationssumpfes aufweist, der aus Edelstahl des Typs 2.4610, 1.4529 oder 1.4539 gefertigt ist.

In einer **einundzwanzigsten Ausführungsform** der Erfindung, die mit allen anderen Ausführungsformen kombiniert werden kann, sofern diese nicht Flüssigphasenphosgenierungen betreffen, erfolgt das Umsetzen des Amins mit Phosgen in Schritt (A) in der Gasphase, wobei (b) umfasst ist.

In einer **zweiundzwanzigsten Ausführungsform** der Erfindung, die eine besondere Ausgestaltung der einundzwanzigsten Ausführungsform ist, wird das Amin ausgewählt aus der Gruppe bestehend aus den Diaminen der Diphenylmethanreihe (sodass die Diisocyanate der Diphenylmethanreihe erhalten werden), Toluylendiamin (sodass Toluylendiisocyanat erhalten wird), Xylylendiamin (sodass Xylylendiisocyanat erhalten wird), Bis(aminomethyl)cyclohexan (sodass Bis(isocyanatomethyl)cyclohexan erhalten wird), Bis(aminomethyl)norbornan (sodass Bis(isocyanatomethyl)norbornan erhahten wird), Hexahydrotoluylendiamin (sodass Hexahydrotoluylendiisocyanat erhalten wird), 1,6-Hexamethylendiamin (sodass Hexamethylendiisocyanat erhalten wird), 1,5-Pentamethylendiamin (sodass Pentamethylendiisocyanat erhalten wird) und Isophorondiamin (sodass Isophorondiisocyanat erhalten wird).

In einer **dreiundzwanzigsten Ausführungsform** der Erfindung, die mit allen anderen Ausführungsformen kombiniert werden kann, sofern diese nicht Gasphasenphosgenierungen betreffen, erfolgt das Umsetzen des Amins mit Phosgen in Schritt (A) in der Flüssigphase, wobei (a) umfasst ist.

In einer **vierundzwanzigsten Ausführungsform** der Erfindung, die eine besondere Ausgestaltung der dreiundzwanzigsten Ausführungsform ist, wird das Amin ausgewählt aus der Gruppe bestehend aus den Di- und Polyaminen der Diphenylmethanreihe (sodass die Di- und Polyisocyanate der Diphenylmethanreihe erhalten werden), Naphthalindiamin (sodass Naphthalindiisocyanat erhalten wird), Toluylendiamin (sodass Toluylendiisocyanat erhalten wird), Xylylendiamin (sodass Xylylendiisocyanat erhalten wird), Bis(aminomethyl)cyclohexan (sodass Bis(isocyanatomethyl)cyclohexan erhalten wird), Bis(aminomethyl)norbornan (sodass Bis(isocyanatomethyl)norbornan erhalten wird), Hexahydrotoluylendiamin (sodass Hexahydrotoluylendiisocyanat erhalten wird), 4,4'-Diaminodicyclohexylmethan (so dass 4,4`-Diisocyanatodicyclohexylmethan erhalten wird), 1,6-Hexamethylendiamin (sodass Hexamethylendiisocyanat erhalten wird), 1,5-Pentamethylendiamin (sodass Pentamethylendiisocyanat erhalten wird) und Isophorondiamin (sodass Isophorondiisocyanat erhalten wird).

In einer **fünfundzwanzigsten Ausführungsform** der Erfindung, die mit allen Ausführungsformen umfassend eine Flüssig- oder Gasphasenphosgenierung, insbesondere umfassend eine Gasphasenphosgenierung, kombiniert werden kann, wird als Amin Toluylendiamin eingesetzt (sodass Toluylendiisocyanat erhalten wird).

In einer **sechsundzwanzigsten Ausführungsform** der Erfindung, die eine besondere Ausgestaltung der fünfundzwanzigsten Ausführungsform ist, gilt X = 2.

In einer **siebenundzwanzigsten Ausführungsform** der Erfindung, die eine besondere Ausgestaltung der dreiundzwanzigsten Ausführungsform ist, wird als Amin eine Mischung aus den Di- und Polyaminen der Diphenylmethanreihe eingesetzt (sodass Di- und Polyisocyanate der Diphenylmethanreihe erhalten werden).

In einer **achtundzwanzigsten Ausführungsform** der Erfindung, die eine besondere Ausgestaltung der siebenundzwanzigsten Ausführungsform ist, gilt X = 1.

In einer **neunundzwanzigsten Ausführungsform** der Erfindung, die eine weitere besondere Ausgestaltung der einundzwanzigsten Ausführungsform ist, wird das Amin ausgewählt aus der Gruppe bestehend aus Hexamethylendiamin (sodass Hexamethylendiisocyanat erhalten wird), Pentamethylendiamin (sodass Pentamethylendiisocyanat erhalten wird) und Isophorondiamin (sodass Isophorondiisocyanat erhalten wird).

In einer **dreißigsten Ausführungsform** der Erfindung, die eine besondere Ausgestaltung der neunundzwanzigsten Ausführungsform ist, gilt X = 1.

In einer **einunddreißigsten Ausführungsform** der Erfindung, die mit allen anderen Ausführungsformen kombiniert werden kann, wird der Rein-Destillation ein Produktgemisch zugeführt, das aromatisches Lösungsmittel der Formel C₆H_{6-X}Cl_{X} in einem auf seine Gesamtmasse bezogenen Massenanteil im Bereich von 8 % bis 49 % enthält.

In einer **zweiunddreißigsten Ausführungsform** der Erfindung, die mit allen anderen Ausführungsformen kombiniert werden kann, wird der Rein-Destillation ein Produktgemisch zugeführt, das aromatisches Lösungsmittel der Formel C₆H_{6-X}Cl_{X} in einem auf seine Gesamtmasse bezogenen Massenanteil im Bereich von 10 % bis 30 %, enthält.

In einer **dreiunddreißigsten Ausführungsform** der Erfindung, die mit allen anderen Ausführungsformen kombiniert werden kann, enthält der ausgeschleuste Strom umfassend aromatisches Lösungsmittel der Formel C₆H_{6-Y}Cl_{Y} dieses (d. h. das aromatische Lösungsmittel der Formel C₆H_{6-Y}Cl_{Y}) in einem auf seine Gesamtmasse (d. h. die Gesamtmasse des ausgeschleusten Stroms) bezogenen Massenanteil im Bereich von 1,0 % bis 10 %.

In einer **vierunddreißigsten Ausführungsform** der Erfindung, die mit allen anderen Ausführungsformen kombiniert werden kann, enthält der ausgeschleuste Strom umfassend aromatisches Lösungsmittel der Formel C₆H_{6-Y}Cl_{Y} dieses (d. h. das aromatische Lösungsmittel der Formel C₆H_{6-Y}Cl_{Y}) in einem auf seine Gesamtmasse (d. h. die Gesamtmasse des ausgeschleusten Stroms) bezogenen Massenanteil im Bereich von 1,5 % bis 5,0 %.

In einer **fünfunddreißigsten Ausführungsform** der Erfindung, die mit allen anderen Ausführungsformen kombiniert werden kann, enthält der ausgeschleuste Strom umfassend aromatisches Lösungsmittel der Formel C₆H_{6-Y}Cl_{Y} dieses (d. h. das aromatische Lösungsmittel der Formel C₆H_{6-Y}Cl_{Y}) in einem auf seine Gesamtmasse (d. h. die Gesamtmasse des ausgeschleusten Stroms) bezogenen Massenanteil im Bereich von 2,2 % bis 3,0 %.

Die zuvor kurz geschilderten und weitere mögliche Ausführungsformen der Erfindung werden im Folgenden näher erläutert. Dabei können alle Ausführungsformen beliebig miteinander kombiniert werden, sofern nichts anderes gesagt wird oder sich aus dem Zusammenhang eindeutig ergibt.

**Schritt (A)** des erfindungsgemäßen Verfahrens, das *Umsetzen eines Amins mit einem stöchiometrischen Überschuss an Phosgen* unter Erhalt *eines flüssigen Produktgemisches umfassend das Isocyanat und das eingesetzte aromatische Lösungsmittel* (neben einem gasförmigen Produktgemisch enthaltend Phosgen und Chlorwasserstoff), kann im Rahmen der vorliegenden Erfindung durchgeführt werden wie aus dem Stand der Technik grundsätzlich bekannt. Wie eingangs geschildert, gibt es die beiden grundsätzlichen Verfahrensvarianten der Flüssig- und der Gasphasenphosgenierung. Die Flüssigphasenphosgenierung erfolgt stets in Gegenwart eines Lösungsmittels als Verdünnungsmittel (*Variante (a)*). Aber auch bei der Gasphasenphosgenierung kann die Reaktion in Gegenwart der Dämpfe eines Lösungsmittels als Verdünnungsmittel erfolgen. Die Zugabe eines Lösungmittels (oder eines Gemisches enthaltend neben dem Lösungsmittel Anteile des gewünschten Isocyanats) zur raschen Abkühlung des Reaktionsgemisches (*Variante (b)*) wird bevorzugt bei Reaktionsführung in der Gasphase angewandt.

Die Umsetzung des primären Amins mit Phosgen in Schritt (A) erfolgt, unabhängig von der Verfahrensführung, bevorzugt kontinuierlich.

Beispiele für **Flüssigphasenphosgenierungen** sind in DE 37 44 001 C1, EP 0 314 985 A1, EP 1369 412 A1, DE-A-102 60 027, DE-A-102 60 093, DE-A 103 10 888, DE-A-10 2006 022 448, US-A 2007/0299279 und der dort jeweils zitierten Literatur beschrieben. Die Flüssigphasenphosgenierung umfasst in Schritt (A) die Variante (a). Variante (b), die sog. Quenche, ist im Allgemeinen nicht erforderlich.

Mit dem erfindungsgemäßen Verfahren können bevorzugt Amine ausgewählt aus den Di- und Polyaminen der Diphenylmethanreihe (sodass die Di- und Polyisocyanate der Diphenylmethanreihe erhalten werden), Naphthalindiamin (sodass Naphthalindiisocyanat erhalten wird), Toluylendiamin (sodass Toluylendiisocyanat erhalten wird), Xylylendiamin (sodass Xylylendiisocyanat erhalten wird), Bis(aminomethyl)cyclohexan (sodass Bis(isocyanatomethyl)cyclohexan erhalten wird), Bis(aminomethyl)norbornan (sodass Bis(isocyanatomethyl)norbornan erhalten wird), Hexahydrotoluylendiamin (sodass Hexahydrotoluylendiisocyanat erhalten wird), 4,4'-Diaminodicyclohexylmethan (so dass 4,4`-Diisocyanatodicyclohexylmethan erhalten wird) 1,6-Hexamethylendiamin (sodass Hexamethylendiisocyanat erhalten wird), 1,5-Pentamethylendiamin (sodass Pentamethylendiisocyanat erhalten wird) und Isophorondiamin (sodass Isophorondiisocyanat erhalten wird) phosgeniert werden. Besonders bevorzugt sind Toluylendiamin sowie die Di- und Polyamine der Diphenlymethanreihe, insbesondere bevorzugt sind die Di- und Polyamine der Diphenylmethanreihe. Dazu wird ein Gemisch aus den Di- und Polyaminen der Diphenylmethanreihe in an sich bekannter Weise zu einem entsprechenden Gemisch aus den Di- und Polyisocyanaten der Diphenylmethanreihe (= MDI) phosgeniert, wobei bevorzugt Monochlorbenzol (X = 1) als Lösungsmittel in Schritt (A) eingesetzt wird (Dichlorbenzol (X = 2) ist jedoch grundsätzlich auch anwendbar). Die Aufarbeitung (Schritt (B)) des in Schritt (A) erhaltenen Produktgemisches umfasst im Fall des MDI bevorzugt auch einen Schritt der Abtrennung einer Fraktion an *Di*isocyanaten der Diphenylmethanreihe (= MMDI; "Monomer-MDI") unter Verbleib eines an *Di*isocyanaten der Diphenylmethanreihe abgereicherten MDI-Gemisches, woran sich eine weitere Rein-Destillation der abgetrennten Monomer-MDI-Fraktion anschließt (im Fall des verbleibenden an Diisocyanaten der Diphenylmethanreihe abgereicherten MDI-Gemisches ist die Abtrennung des Monomer-MDI als "Rein-Destillation" zu betrachten). Die erfindungsgemäßen Anforderungen an den Restgehalt an diesem Lösungsmittel im Produktstrom beziehen sich auf das an Monomer-MDI abgereicherte MDI-Gemisch und das isolierte MMDI selbst.

In einer bevorzugten Ausgestaltung der Flüssigphasenphosgenierung wird wie folgt verfahren:
Die Reaktionspartner primäres Amin und Phosgen werden getrennt voneinander in einem Lösungsmittel gelöst. Geeignete Lösungsmittel für diesen Zweck sind Monochlorbenzol (X = 1) und Dichlorbenzol (X = 2), Letzteres in Form des ortho-oder para-Isomers, bevorzugt des ortho-Isomers. Das primäre Amin wird bevorzugt in einer Konzentration von 10 Massen-% bis 40 Massen-%, bevorzugt von 10 Massen-% bis 20 Massen-%, bezogen auf die Gesamtmasse der Lösung, verwendet. Phosgen wird bevorzugt in einer Konzentration von 10 Massen-% bis 40 Massen-%, bevorzugt von 25 Massen-% bis 35 Massen-%, bezogen auf die Gesamtmasse der Lösung, verwendet.

Eine effiziente Vermischung von primärem Amin und Phosgen ist beim Flüssigphasenverfahren von hoher Bedeutung. Hierzu kommen im Stand der Technik statische (bevorzugt Düsen) und dynamische (enthaltend mechanisch bewegte Teile) Mischeinrichtungen zum Einsatz. Nach der Vermischung durchlaufen die vermischten Reaktionspartner eine Reaktionszone zur Umsatzvervollständigung. Mischeinrichtung und Reaktionszone können auch in einem gemeinsamen Reaktor angeordnet sein. Phosgen wird im stöchiometrischen Überschuss gegenüber den primären Aminogruppen des Amins eingesetzt, insbesondere in einem molaren Verhältnis von Phosgen zu primären Aminogruppen im Bereich von 4,0 : 1 bis 1,1 : 1, besonders bevorzugt im Bereich von 3,0 : 1 bis 1,1 : 1, ganz besonders bevorzugt im Bereich von 2,0 : 1 bis 1,1 : 1.

Die Flüssigphasenphosgenierung kann bei verschiedenen Temperatur- und Druckniveaus durchgeführt werden. So ist es beispielsweise möglich, die Flüssigphasenphosgenierung bei einer Temperatur im Bereich von 0 °C bis 250 °C, bevorzugt von 20 °C bis 200 °C und bei einem Druck im Bereich von 1,0 bar_{(abs.)} bis 70 bar_{(abs.)}, bevorzugt von 1,0 bar_{(abs.)} bis 50 bar_{(abs.)}, durchzuführen.

In einer bevorzugten Ausführungsform liegt der bei der Reaktion als Koppelprodukt entstehende Chlorwasserstoff zum Teil gelöst in der flüssigen Phase vor und gast zum Teil aus. Wie groß der Anteil des gelösten gegenüber dem gasförmig vorliegenden Chlorwasserstoff ist, hängt vom gewählten Temperatur- und Druckniveau ab.

In einer anderen Ausführungsform werden das Temperatur- und Druckniveau so gewählt, dass Chlorwasserstoff zunächst im Wesentlichen komplett gelöst oder verflüssigt vorliegt und sich eine Gasphase erst nach einer gezielten Entspannung (zum Beispiel in einem Gas-/Flüssigkeitsabscheider) ausbildet. Eine solche Entspannung ist in der Terminologie der vorliegenden Erfindung Bestandteil des Schrittes (A).

Daher werden in jedem Fall am Ende von Schritt (A) ein flüssiger, das herzustellende Isocyanat und Lösungsmittel enthaltender Strom und ein gasförmiger, Chlorwasserstoff und gegebenenfalls verdampftes Lösungsmittel enthaltender Strom erhalten. Da Phosgen überstöchiometrisch eingesetzt wird, enthalten beide Ströme zudem Phosgen. Beide Ströme können direkt der Reaktionszone entnommen werden. Es ist auch möglich, der Reaktionszone ein zweiphasiges Verfahrensprodukt (enthaltend eine Flüssig- und eine Gasphase) zu entnehmen und dieses in eine Vorrichtung zur Phasentrennung zu überführen. Diese Phasentrennung kann allen dem Fachmann bekannten Apparaten, die zur Trennung von Gas- und Flüssigphasen geeignet sind, erfolgen. Bevorzugt werden Gas- und Flüssigkeitsabscheider wie z. B. Zyklonabscheider, Umlenkabscheider und/oder Schwerkraftabscheider mit und ohne statische Abscheidehilfe eingesetzt. Es ist ebenfalls möglich, die Phasentrennung durch Verminderung des Drucks gegenüber dem in der Reaktionszone herrschendem Druck infolge verstärkten Ausgasens von Chlorwasserstoff (und gegebenenfalls anderer gasförmiger Bestandteile) zu unterstützen. Der der Reaktionszone entnommene flüssige Strom bzw. - sofern vorhanden - der dem der Reaktionszone nachgeschalteten Apparat zur Phasentrennung entnommene flüssige Strom ist in dieser Ausführungsform das Ausgangsmaterial der in Schritt (B) durchzuführenden Aufarbeitung, d. h. diese Flüssigphase ist in der Terminologie der vorliegenden Erfindung das *"flüssige Produktgemisch umfassend das Isocyanat und das eingesetzte aromatische Lösungsmittel".*

Am Beispiel des primären Amins TDA wird die Flüssigphasenphosgenierung im Folgenden noch näher geschildert:
Im Flüssigphasenverfahren wird das TDA, in einem der weiter oben definierten Lösungsmittel gelöst, der Vermischung mit Phosgen bei einer Temperatur im Bereich von -10 °C bis 220 °C, bevorzugt von 0° C bis 200 °C, besonders bevorzugt von 20 °C bis 180 °C, zugeführt. Das Phosgen wird der Vermischung mit TDA ebenfalls in einem der weiter oben definierten Lösungsmittel gelöst mit einer Temperatur im Bereich von -40 °C bis 200 °C, bevorzugt von -30 °C bis 170 °C, besonders bevorzugt von -20 °C bis 150 °C zugeführt. Die Vermischung der TDA- und Phosgen-Lösungen erfolgt im Flüssigphasenverfahren bevorzugt mittels eines statischen Mischers oder eines dynamischen Mischers. Beispiele für geeignete statische Mischer sind u. a. Düsen bzw. Düsenanordnungen, wie z. B. in DE 17 92 660 A, US 4,289,732 oder US 4,419,295 beschrieben. Beispiele für geeignete dynamische Mischer sind u. a. pumpenähnliche Aggregate, wie beispielsweise Kreiselpumpen (vgl. US 3,713,833) oder spezielle Mischer-Reaktoren (vgl. EP 0 291 819 A, EP 0 291 820 A, EP 0 830 894 A).

Die Umsetzung in der sich anschließenden Reaktionszone erfolgt im Flüssigphasenverfahren bei einer Temperatur im Bereich von 0 °C bis 250 °C, bevorzugt von 20 °C bis 200 °C, besonders bevorzugt von 20 °C bis 180 °C, mit einer mittleren Verweilzeit des Reaktionsgemisches in der Reaktionszone im Bereich von 10 s bis 5 h, bevorzugt von 30 s bis 4 h, besonders bevorzugt von 60 s bis 3 h, und bei einem Druck von bis zu 100 bar_{(abs.)}, bevorzugt im Bereich von 1,0 bar_{(abs.)} bis 70 bar_{(abs.)}, besonders bevorzugt von 1,0 bar_{(abs.)} bis 50 bar_{(abs.)}. Beispiele für erfindungsgemäß einsetzbare Verfahrensführungen im Hinblick auf die Umsetzung in der Reaktionszone sind z. B. in US-A 2007/0299279 (insbesondere S. 7, Abschnitte [0070], [0071], [0089]) und DE-A 103 10 888 (insbesondere S. 5 Abschnitte [0038], [0039]) und den darin jeweils zitierten Schriften beschrieben.

Beispiele für **Gasphasenphosgenierungen** sind in EP 0 570 799 A1, EP 1 555 258 A1, EP 1 526 129 A1 und DE 101 61 384 A1, sowie insbesondere für aliphatische Isocyanate in EP 0 289 840 B1, EP 1 754 698 B1, EP 1 319 655 B1 und EP 1 362 847 B1 beschrieben. Vorteile dieses Verfahrens gegenüber der ansonsten üblichen Flüssigphasenphosgenierung liegen in der Energieeinsparung, bedingt durch die Minimierung eines aufwändigen Lösungsmittel- und Phosgenkreislaufs. Die Gasphasenphosgenierung umfasst in Schritt (A) die Variante (b). Variante (a), im Fall der Gasphasenphosgenierung der Einsatz von Dämpfen des in Schritt (A) eingesetzten aromatischen Lösungsmittels als Verdünnungsmittels, ist ebenfalls möglich, aber nicht zwingend.

Mit dem erfindungsgemäßen Verfahren können bevorzugt Amine ausgewählt aus aus den Diaminen der Diphenylmethanreihe (sodass die Diisocyanate der Diphenylmethanreihe erhalten werden), Toluylendiamin (sodass Toluylendiisocyanat erhalten wird), Xylylendiamin (sodass Xylylendiisocyanat erhalten wird), Bis(aminomethyl)cyclohexan (sodass Bis(isocyanatomethyl)cyclohexan erhalten wird), Bis(aminomethyl)norbornan (sodass Bis(isocyanatomethyl)norbornan erhahten wird), Hexahydrotoluylendiamin (sodass Hexahydrotoluylendiisocyanat erhalten wird), 1,6-Hexamethylendiamin (sodass Hexamethylendiisocyanat erhalten wird), 1,5-Pentamethylendiamin (sodass Pentamethylendiisocyanat erhalten wird) und Isophorondiamin (sodass Isophorondiisocyanat erhalten wird). Besonders bevorzugt ist Toluylendiamin, wobei vorzugsweise Dichlorbenzol (X = 2), insbesondere das ortho-Isomer, als Lösungsmittel in Schritt (A) eingesetzt wird. Dazu wird Toluylendiamin, das als ein Gemisch aus verschiedenen Isomeren, insbesondere 2,4- und 2,6-Toluylendiamin (meta-Toluylendiamin) vorliegt, in an sich bekannter Weise zu einem entsprechenden Gemisch aus den Toluylendiisocyanat-Isomeren (= TDI) phosgeniert, wobei wie bereits erwähnt bevorzugt Dichlorbenzol (X = 2), insbesondere das ortho-Isomer, als Lösungsmittel in Schritt (A) eingesetzt wird (Monochlorbenzol (X = 1) ist jedoch grundsätzlich auch anwendbar).

In einer bevorzugten Ausgestaltung der Gasphasenphosgenierung wird wie folgt verfahren:
Zunächst wird ein gasförmiger Strom eines primären Amins bereitgestellt. Geeignete Methoden hierfür sind dem Fachmann grundsätzlich bekannt. Im Folgenden werden bevorzugte Ausführungsformen geschildert.

Die Überführung des primären Amins in die Gasphase kann in allen aus dem Stand der Technik bekannten Verdampfungsapparaturen erfolgen, insbesondere in einem Fallfilmverdampfer. Bevorzugt werden solche Verdampfungsapparaturen eingesetzt, bei denen ein kleiner Arbeitsinhalt mit einer hohen Umwälzleistung über einen Fallfilmverdampfer geführt wird.

Zur Minimierung der thermischen Belastung des Amins ist es unabhängig von der genauen Ausgestaltung der Verdampfungsapparatur bevorzugt, den Verdampfungsvorgang durch Zuspeisung eines Inertgases wie N₂, He, Ar (insbesondere N₂) oder der Dämpfe eines Lösungsmittels zu unterstützen. Geeignete Lösungsmittel für diesen Zweck sind Monochlorbenzol (X = 1) und Dichlorbenzol (X = 2), Letzteres in Form des ortho-oder para-Isomers, bevorzugt des ortho-Isomers.

Die Verdampfung - und erforderlichenfalls Überhitzung - des Ausgangsamins (insbesondere auf eine Temperatur im Bereich von 200 °C bis 430 °C, bevorzugt 250 °C bis 420 °C, besonders bevorzugt 250 °C bis 400 °C) erfolgt ein- oder mehrstufig, bevorzugt mehrstufig, um nicht verdampfte Tröpfchen im gasförmigen Aminstrom so weit wie möglich zu vermeiden, mehrstufig. Insbesondere bevorzugt sind mehrstufige Verdampfungs- und Überhitzungsschritte, in denen zwischen den Verdampfungs- und Überhitzungssystemen Tröpfchenabscheider eingebaut sind (an mindestens einer Stelle, d. h. zwischen mindestens einem Verdampfungssystem und dem nachfolgenden Überhitzungssystem, oder auch zwischen jedem Verdampfungssystem und dem nachfolgenden Überhitzungssystem) und / oder die Verdampfungsapparaturen auch die Funktion eines Tröpfchenabscheiders besitzen. Geeignete Tröpfchenabscheider sind dem Fachmann bekannt.

In einem weiteren Schritt wird ein gasförmiger Phosgenstrom bereitgestellt. Bevorzugt wird ein molares Verhältnis von Phosgen zu primären Amingruppen von 1,1 : 1 bis 20 : 1, besonders bevorzugt 1,2 : 1 bis 5,0 : 1 eingestellt. Auch das Phosgen wird bevorzugt, wie zuvor für das primäre Amin beschrieben, auf eine Temperatur im Bereich von 200 °C bis 430 °C, bevorzugt 250 °C bis 420 °C, besonders bevorzugt 250 °C bis 400 °C, erhitzt und gegebenenfalls mit einem Inertgas wie N₂, He, Ar (insbesondere N₂) oder mit den Dämpfen eines inerten Lösungsmittels wie zuvor für das Amin definiert verdünnt.

Die gasförmig vorliegenden Reaktionspartner primäres Amin und Phosgen werden in einer Mischzone vermischt und in einer sich an diese anschließenden Reaktionszone umgesetzt. Die getrennt aufgeheizten Reaktionspartner Amin und Phosgen werden bevorzugt über eine Düsenanordnung der Vermischung und Umsetzung zugeführt. Die Düsenanordnung zur Einführung der Eduktgasströme Amin und Phosgen kann auf verschiedene dem Fachmann bekannte Arten ausgestaltet sein; Beispiele finden sich z. B. in EP 2 199 277 B1, Abschnitt [0017] bis [0019], EP 1 449 826 B1, Abschnitt [0011] bis [0012], EP 1 362 847 B1, Abschnitt [0011] bis [0012], EP 1 526 129 B1, Abschnitt [0009] bis [0011] und EP 1 555 258 B1, Abschnitt [0008] bis [0011].

Neben der bereits erwähnten Möglichkeit, den gasförmigen Strom des primären Amins und den gasförmigen Phosgenstrom zu verdünnen, kann auch ein separater Verdünnungsgasstrom (ein Inertgas wie N₂, He, Ar (insbesondere N₂) oder die Dämpfe eines inerten Lösungsmittels wie zuvor für das Amin definiert direkt in die Vermischung gefahren werden. In diesem Fall wird dieser Verdünnungsgasstrom bevorzugt auf eine Temperatur im Bereich von 100 °C bis 500 °C, bevorzugt 150 °C bis 450 °C, besonders bevorzugt 150 °C bis 400 °C erhitzt.

Die weitere Umsetzung der in der Mischzone vermischten Reaktionspartner primäres Amin und Phosgen in der Reaktionszone erfolgt bevorzugt adiabatisch. Adiabatische Umsetzung bedeutet, dass auf eine gezielte Abfuhr der entstandenen Reaktionswärme durch ein Wärmeträgermedium verzichtet wird. Daher spiegelt sich die Reaktionsenthalpie - abgesehen von unvermeidbaren Wärmeverlusten - quantitativ in der Temperaturdifferenz von Produkt- und Eduktgasstrom wider.

In der Reaktionszone werden Amin und Phosgen rasch zum korrespondierenden Isocyanat umgesetzt, und zwar bevorzugt adiabatisch. Die Reaktion wird bevorzugt so geführt, dass das Amin vor Eintritt in nachfolgend näher beschriebene *Quenche* vollständig umgesetzt ist.

In der sog. Quenche erfolgt in einer Quenchzone eine rasche Abkühlung und (bis auf Spurenanteile, die in der Gasphase verbleiben) Verflüssigung des gebildeten Isocyanats ("Quenche") durch Inkontaktbringen mit einer Quenchflüssigkeit in einer Quenchzone. Als Quenchflüssigkeit eignen sich die oben genannten Lösungsmittel und Gemische aus dem herzustellenden Isocyanat und den genannten Lösungsmitteln. Das Inkontaktbringen erfolgt bevorzugt durch Eindüsen der Quenchflüssigkeit in den gasförmigen Strom des Reaktionsproduktgemisches. Möglichkeiten für den Aufbau und den Betrieb einer Quenchzone sind im Prinzip aus dem Stand der Technik bekannt. Die Apparate und Methoden des Standes der Technik können auch im Rahmen der vorliegenden Erfindung eingesetzt werden. Mögliche Ausgestaltungen der Quenchzone sind beispielsweise in EP 1 403 248 A1 und EP 1 935 875 A1 offenbart. Die Temperatur der in der Quenche eingesetzten Quenchflüssigkeit wird bevorzugt so gewählt, dass sie einerseits hoch genug ist, um das dem Isocyanat entsprechende Carbamoylchlorid in Isocyanat und Chlorwasserstoff zurückzuspalten. (Es ist zwar keineswegs sicher, ob das aus der Flüssigphasenphosgenierung bekannte Zwischenprodukt Carbamoylclorid auch in der Gashasenphosgenierung gebildet wird. Da es jedoch unabhängig davon denkbar ist, dass in der Quenche *verflüssigtes* Isocyanat mit dem vorhandenem Chlorwasserstoffgas teilweise zum Carbamoylchlorid reagiert, sollte die Temperatur der Quenchflüssigkeit hoch genug sein, um diese Reaktion zurückzudrängen.) Andererseits sollen Isocyanat und gegebenenfalls das in dem gasförmigen Aminstrom und / oder gasförmigen Phosgenstrom als Verdünnungsmittel mit verwendete Lösungsmittel weitestgehend kondensieren bzw. sich weitestgehend in dem Lösungsmittel lösen, während überschüssiges Phosgen, Chlorwasserstoff und gegebenenfalls als Verdünnungsmittel mit verwendetes Inertgas die Quenchzone weitestgehend nicht kondensiert bzw. nicht gelöst durchlaufen, sodass die Temperatur der Quenchflüssigkeit auch nicht zu hoch gewählt werden darf. Zur selektiven Gewinnung des Isocyanats aus dem gasförmigen Reaktionsgemisch besonders gut geeignet sind bei einer Temperatur von 50 °C bis 200 °C, vorzugsweise 80 C bis 180 °C gehaltene Quenchflüssigkeiten. Das in der Quenchzone erhaltene Gemisch enthält gasförmige Anteile und flüssige Anteile, ist also zweiphasig. Dieses zweiphasige Gemisch wird in eine *Sammelzone* zur Phasentrennung geführt. Die flüssige und gasförmige Phase werden der Sammelzone bevorzugt kontinuierlich entnommen. Die so erhaltende Flüssigphase ist in dieser Ausführungsform das Ausgangsmaterial der in Schritt (B) durchzuführenden Aufarbeitung, d. h. diese Flüssigphase ist das *flüssige Produktgemisch umfassend das Isocyanat und das eingesetzte aromatische Lösungsmittel.*

In einer bevorzugten Ausführungsform sind Mischzone, Reaktionszone, Quenchzone und Sammelzone von oben nach unten in der genannten Reihenfolge in einem aufrecht stehenden, insbesondere konischen oder zylindrischen oder konisch-zylindrischen, Reaktor angeordnet. In dieser Ausführungsform fließt das in der Quenche anfallende Gemisch schwerkraftbedingt in die Sammelzone. Bei anderer Anordnung der Sammelzone ist es unter Umständen erforderlich, das Gemisch aus Reaktionsproduktgemisch und Quenchflüssigkeit in die Sammelzone zu pumpen.

Nach der Phosgenierung in Schritt (A) erfolgt in **Schritt (B)** die Aufarbeitung des flüssigen Produktgemisches aus (A), also das *Isolieren des Isocyanats aus dem in Schritt (A) erhaltenen flüssigen Produktgemisch* unter Erhalt des Isocyanats *mit einem auf seine Gesamtmasse bezogenen Massenanteil an aromatischem Lösungsmittel der Formel C₆H_{6-Y}Cl_{Y} im Bereich von 0,0 ppm bis 9,9 ppm, bevorzugt 0,0 ppm bis 5,0 ppm, besonders bevorzugt 0,0 ppm bis 3,0 ppm.* Schritt (B) umfasst erfindungsgemäß mindestens den Schritt einer Rein-Destillation und bevorzugt mindestens einen der Rein-Destillation vorgelagerten Destillationsschritt, insbesondere eine Lösungsmittel-Destillation zur Abtrennung von aromatischem Lösungsmittel der Formel C₆H_{6-X}Cl_{X}. In bestimmten Ausführungsformen umfasst Schritt (B) auch eine Entphosgenierung zur Abtrennung gelösten Phosgens aus dem in Schritt (A) erhaltenen flüssigen Produktgemisch, wobei die Entphosgenierung nicht zwingend als Destillation ausgestaltet werden muss (siehe unten). In bestimmten Fällen kann es auch erforderlich sein, der Rein-Destillation einen eigenen Destillationsschritt zur Abtrennung von sog. Rückstand (sehr hoch siedende Nebenprodukte der Phosgenierung) voranzustellen (Rückstandsabtrennung). Die hier nur kurz umrissenen Schritte werden weiter unten näher erläutert.

Bevorzugt werden der Schritt der Rein-Destillation und, sofern durchgeführt, die diesem vorgelagerten Destillationsschritte, *kontinuierlich* durchgeführt, sodass kontinuierlich ein Strom isolierten Isocyanats anfällt. Der *Massenanteil an aromatischem Lösungsmittel der Formel C₆H_{6-Y}Cl_{Y}* wird dann in diesem kontinuierlich anfallenden Strom isolierten Isocyanats bestimmt, und zwar insbesondere in Intervallen von 1 Stunde bis 16 Stunden, bevorzugt von 4 Stunden bis 12 Stunden. Bei *diskontinuierlicher* Ausgestaltung des Schrittes (B) fällt das isolierte Isocyanat diskontinuierlich, also chargenweise an. Der *Massenanteil an aromatischem Lösungsmittel der Formel C₆H_{6-Y}Cl_{Y}* wird dann vorzugsweise von jeder Charge bestimmt. Sind die Betriebsbedingungen für Schritt (B) einmal auf einen hinreichend geringen Massenanteil an aromatischem Lösungsmittel der Formel C₆H_{6-Y}Cl_{Y} optimiert, kann die Messhäufigkeit auch verringert werden, unabhängig davon, ob das isolierte Isocyanat kontinuierlich oder chargenweise anfällt.

Ebenfalls unabhängig davon, ob das isolierte Isocyanat kontinuierlich oder chargenweise anfällt, wird bei einer festgestellten Abweichung vom erfindungsgemäß vorgesehenen *Massenanteil an aromatischem Lösungsmittel der Formel C₆H_{6-Y}Cl_{Y}* gegengesteuert, indem aromatisches Lösungsmittel der Formel C₆H_{6-Y}Cl_{Y} verstärkt ausgeschleust wird.

Beispiele für die Aufarbeitung von Phosgenierungsprodukten sind in EP-A-1 413 571 (TDI), US 2003/0230476 A1 (TDI), und EP 0289 840 B1 (HDI, IDPI und H12-MDI) beschrieben. Die dort beschriebenen *grundsätzlichen Verfahrensweisen* können prinzipiell auch im erfindungsgemäßen Verfahren Anwendung finden. Dies gilt, sofern die erfindungsgemäß geforderte Ausschleusung mindestens eines Stroms umfassend einfach zusätzlich chloriertes aromatisches Lösungsmittel C₆H_{6-Y}Cl_{Y} in diese Verfahrensweisen so eingefügt werden kann, dass die Reinheitsanforderungen in Bezug auf die Konzentration an C₆H_{6-Y}Cl_{Y} im angestrebten Isocyanat eingehalten werden.

Wie bereits weiter oben erwähnt wird zunächst optional gelöstes Phosgen (sowie gelöster Chlorwasserstoff) aus dem in Schritt (A) erhaltenen flüssigen Produktgemisch in einem separaten Schritt abgetrennt. Diese Verfahrensführung ist insbesondere dann bevorzugt, wenn die Phosgenierung in Schritt (A) in der Flüssigphase durchgeführt wird, weil das in einer Flüssigphasenphosgenierung erhaltene flüssige rohe Verfahrensprodukt tendenziell signifikant höhere Anteile an gelöstem Phosgen und gelöstem Chlorwasserstoff enthält als das in einer Gasphasenphosgenierung erhaltene. Dieser sog. *Entphosgenierungsschritt* kann grundsätzlich in jeder dem Fachmann bekannten Art und Weise durchgeführt werden, insbesondere durch Destillation, Absorption oder eine Kombination beider.

Im Anschluss an den Entphosgenierungsschritt oder - insbesondere bei Durchführung des Schritts a) in der Gasphase - unmittelbar im Anschluss an Schritt (A), schließt sich, wie oben bereits erwähnt, bevorzugt eine Abtrennung von eingesetztem aromatischem Lösungsmittel C₆H_{6-X}Cl_{X} (genauer: eines Hauptteils desselben, nämlich so viel, dass ein an aromatischem Lösungsmittel der Formel C₆H_{6-X}Cl_{X} abgereichertes flüssiges Produktgemisch erhalten wird, dessen auf seine Gesamtmasse bezogener Massenanteil an aromatischem Lösungsmittel der Formel C₆H_{6-X}Cl_{X} im Bereich von 8 % bis 49 %, bevorzugt 10 % bis 30 %, liegt) in einem separaten Schritt an. Diese *Lösungsmittelabtrennung* erfolgt durch Destillation (*Lösungsmittel-Destillation*)*.* Das dabei abgetrennte Lösungsmittel kann erforderlichenfalls einer *Lösungsmittel-Reinigung* zur Abtrennung von darin enthaltenem Phosgen (= Lösungsmittel-Entphosgenierung) unterzogen werden. Dies geschieht vorzugsweise durch Destillation in einer Lösungsmittel-Entphosgenierkolonne, wobei als Sumpfprodukt gereinigtes Lösungsmittel anfällt, das Phosgen und Isocyanat allenfalls noch in Spuren enthält. In einer solchen zur Entphosgenierung durchgeführten Destillation kann jedoch keine Trennung des eingesetzten vom einfach zusätzlich chlorierten aromatischen Lösungsmittel stattfinden, da beide gemeinsam im Sumpf der Entphosgenierkolonne anfallen. Aus wirtschaftlichen Gründen ist es angestrebt, zurückgewonnenes, gegebenenfalls entphosgeniertes Lösungsmittel nach Schritt (A) zurückzuführen. Zurückgewonnenes, gegebenenfalls entphosgeniertes Lösungsmittel der Formel C₆H_{6-X}Cl_{X} kann auch Anteile des höher siedenden Lösungsmittels der Formel C₆H_{6-Y}Cl_{Y} enthalten (während andere Anteile desselben im an Lösungsmittel der Formel C₆H_{6-X}Cl_{X} abgereicherten flüssigen Produktgemisch aus dem Sumpf der Lösungsmittel-Destillation verbleiben). Ist dies der Fall, fällt also das Lösungsmittel der Formel C₆H_{6-X}Cl_{X} in der Lösungsmitteldestillation in einem Gemisch mit Lösungsmittel der Formel C₆H_{6-Y}Cl_{Y} an, so kann in einer Ausführungsform der Erfindung die Gesamtheit dieses Gemisches nach Schritt (A) zurückgeführt werden. In diesem Fall verlagert sich dann die erfindungswesentliche *Ausschleusung eines Stroms umfassend aromatisches Lösungsmittel der Formel C₆H_{6-Y}Cl_{Y}* vollständig auf den nachgelagerten Schritt der Feinreinigung (= Rein-Destillation) des Isocyanats. Es ist jedoch auch möglich, diese erfindungswesentliche Ausschleusung zumindest zum Teil bereits im Schritt der Lösungsmittelabtrennung durchzuführen. Dazu geeignete Ausführungsformen werden im Folgenden beschrieben:

In einer derartigen Ausführungsform der Erfindung wird also die Lösungsmittel-Destillation so ausgestaltet, dass das in der Lösungsmittel-Destillation abgetrennte aromatische Lösungsmittel der Formel C₆H_{6-X}Cl_{X} in einem Gemisch mit aromatischem Lösungsmittel der Formel C₆H_{6-Y}Cl_{Y} erhalten wird, wobei ein erster Teil des Gemisches (optional nach Lösungsmittel-Entphosgenierung; siehe oben) in Schritt (A) zurückgeführt und ein zweiter Teil des Gemisches nicht in Schritt (A) zurückgeführt, sondern (in Intervallen oder kontinuierlich) ausgeschleust und anschließend verbrannt oder anderweitig eingesetzt, jedoch nicht wieder in das Verfahren zurückgeführt wird. Die Verbrennung ist bevorzugt. In dieser Ausführungsform Erfindung stellt die beschriebene Abtrennung des zweiten Teils des Gemisches (unter bewusstem Verzicht auf dessen Rückführung in den Prozess) die erfindungswesentliche *Ausschleusung eines Stroms umfassend aromatisches Lösungsmittel der Formel C₆H_{6-Y}Cl_{Y}* oder einen Teil derselben (wenn es noch weitere Ausschleusungspunkte gibt) dar.

Die entsprechende Ausgestaltung der Lösungsmittel-Destillation erfolgt durch die Einstellung geeigneter Bedingungen von Temperatur, Druck, Rücklaufverhältnis und/oder Zahl der Trennstufen in dieser Destillation. Die genau zu wählenden Bedingungen hängen selbstredend von der Art des in Schritt (A) eingesetzten aromatischen Lösungsmittels, des Amins und der Konzentration sowohl des Isocyanats als auch des einfach zusätzlich chlorierten aromatischen Lösungsmittels in der zu destillierenden Ausgangsmischung ab und können vom Fachmann leicht für jeden Anwendungsfall ermittelt werden. Gegebenenfalls können einfache Vorversuche zur Festlegung der optimalen Destillationsparameter erforderlich sein.

Alternativ zur Ausschleusung *des zweiten Teils* des in der Lösungsmittel-Destillation erhaltenen Gemisches kann der zweite Teil des Gemisches in einer weiteren Destillation (die in der Terminologie der vorliegenden Erfindung als Bestandteil der Lösungsmittel-Destillation und damit als ein der Rein-Destillation vorgelagerter Destillationsschritt gilt) gereinigt werden, wobei aromatisches Lösungsmittel der Formel C₆H_{6-X}Cl_{X} aus dem zweiten Teil des Gemisches abgetrennt und anschließend (optional nach Lösungsmittel-Entphosgenierung) zumindest teilweise, insbesondere vollständig, in Schritt (A) zurückgeführt wird. Es ist selbstredend ebenfalls möglich, *das gesamte* in der Lösungsmittel-Destillation abgetrennte Gemisch in einer weiteren Destillation zu reinigen und dabei aromatisches Lösungsmittel der Formel C₆H_{6-X}Cl_{X} aus dem Gemisch abzutrennen und anschließend (optional nach Lösungsmittel-Entphosgenierung) zumindest teilweise, insbesondere vollständig, in Schritt (A) zurückzuführen.

Unabhängig davon, ob nur der zweite Teil des Gemisches oder dessen Gesamtheit der vorstehend beschriebenen weiteren Destillation zugeführt wird, reichert sich das einfach zusätzlich chlorierte aromatische Lösungsmittel C₆H_{6-Y}Cl_{Y} aufgrund seines vergleichsweise hohen Siedepunktes im Sumpf dieser weiteren Destillation an und verlässt diese daher über den Sumpfstrom, während ein Strom aromatischen Lösungsmittels der Formel C₆H_{6-X}Cl_{X} (der das das einfach zusätzlich chlorierte aromatische Lösungsmittel C₆H_{6-Y}Cl_{Y} allenfalls in unbedeutenden Anteilen enthält) als Kopfstrom anfällt. Der Sumpfstrom dieser weiteren Destillation wird daher ausgeschleust und verbrannt oder anderweitig eingesetzt, jedoch nicht wieder in das Verfahren zurückgeführt. Die Verbrennung ist bevorzugt. In dieser Ausführungsform Erfindung stellt die beschriebene Abtrennung des Sumpfstromes der weiteren Destillation (unter bewusstem Verzicht auf dessen Rückführung in den Prozess) die erfindungswesentliche *Ausschleusung eines Stroms umfassend aromatisches Lösungsmittel der Formel C₆H_{6-Y}Cl_{Y}* oder einen Teil derselben (wenn es noch weitere Ausschleusungspunkte gibt) dar.

Die *Ausschleusung* eines Teils (des zweiten Teils) des in der Lösungsmittel-Destillation erhaltenen Gemisches und/oder die *Reinigung* eines Teils dieses Gemisches bzw. des gesamten Gemisches in einer *weiteren Destillation* gefolgt von der *Ausschleusung des Sumpfstromes* dieser weiteren Destillation sind dabei so vorzunehmen, dass einfach zusätzlich chloriertes aromatisches Lösungsmittel C₆H_{6-Y}Cl_{Y} in hinreichendem Maße aus dem Prozess ausgeschleust wird. Die genauen Bedingungen (wie etwa das Mengenverhältnis des ersten zum zweiten Teil des Gemisches und/oder die genaue Ausgestaltung der weiteren Destillation und/oder die Häufigkeit der Ausschleusung bei Durchführung derselben in Intervallen) sind von den Rahmenbedingungen des Einzelfalls abhängig und können vom Fachmann leicht ermittelt werden, gegebenenfalls unter Durchführung von Vorversuchen. Zu den relevanten Rahmenbedingungen gehört natürlich auch, ob es weitere Stellen im Prozess zur Ausschleusung einfach zusätzlich chlorierten aromatischen Lösungsmittels C₆H_{6-Y}Cl_{Y} gibt.

Die Ausschleusung des einfach zusätzlich chlorierten aromatischen Lösungsmittels in einem für die Zwecke der vorliegenden Erfindung hinreichendem Maße *allein* in der Lösungsmittel-Destillation ist nämlich, obwohl grundsätzlich möglich, aufwändig. Daher ist es bevorzugt, die Ausschleusung des einfach zusätzlich chlorierten aromatischen Lösungsmittels überwiegend oder vollständig im nachfolgend beschriebenen Schritt der *Feinreinigung* vorzunehmen.

Das der Feinreinigung (= *Rein-Destillation*) zuzuführende Produktgemisch enthält insbesondere aromatisches Lösungsmittel der Formel C₆H_{6-X}Cl_{X} in einem auf seine Gesamtmasse bezogenen Massenanteil im Bereich von 8 % bis 49 %, bevorzugt 10 % bis 30 %, unabhängig davon, ob der Feinreinigung eine Lösungsmittelabtrennung vorgeschaltet ist oder nicht (d. h. entweder wird ein in den genannten Bereichen liegender Wert des Lösungsmittelgehalts durch die Wahl der Bedingungen in der Lösungsmittelabtrennung eingestellt oder er stellt sich als Folge der Reaktionsbedingungen in Schritt (A) von selbst ein). Diese Feinreinigung des herzustellenden Isocyanats erfolgt durch Destillation zur Abtrennung der noch vorhandenen leicht- und hochsiedenden organischen Nebenkomponenten (= Leicht- und Hochsieder) und von noch vorhandenem Lösungsmittel sowie gegebenenfalls von noch vorhandenen Restbestandteilen an Chlorwasserstoff und Phosgen. Noch vorhandenes Lösungsmittel, Chlorwasserstoff und Phosgen sieden ebenfalls leichter als das herzustellende Isocyanat und werden daher überwiegend bis vollständig gemeinsam mit den leichtsiedenden organischen Nebenkomponenten abgetrennt. Die Rein-Destillation kann ihrerseits wieder aus Teilschritten bestehen, dergestalt, dass die Abtrennung der Leichtsieder und der Hochsieder in zwei hintereinandergeschalteten Destillationskolonnen erfolgt. Es ist jedoch ebenfalls möglich, diese Trennoperationen in einem Schritt (d. h. in einer einzigen Destillationskolonne) durchzuführen, wobei eine Trennwandkolonne eingesetzt wird.

Schritt (B) umfasst bevorzugt (insbesondere bei Durchführung von Schritt (A) in der Gasphase) auch eine Aufarbeitung des in Schritt (A) erhaltenen *gasförmigen* Produktgemisches zur Abtrennung von darin gegebenenfalls enthaltenem Isocyanat. Dies geschieht vorzugsweise mittels einer Wäsche des gasförmigen Produktgemisches mit eingesetztem aromatischem Lösungsmittel C₆H_{6-X}Cl_{X}. Das zu diesem Zweck eingesetzte Lösungsmittel der Formel C₆H_{6-X}Cl_{X} muss nicht 100%ig rein sein im Hinblick auf einfach zusätzlich chloriertes aromatisches Lösungsmittel C₆H_{6-Y}Cl_{Y}. Das in der Wäsche erhaltene Isocyanat-Lösungsmittelgemisch kann in die Aufarbeitung des flüssigen Produktgemisches aus Schritt (A) geführt werden.

Nachfolgend werden am Beispiel eines besonders bevorzugten Isocyanats, dem TDI, mögliche Ausführungsformen für Schritt (B) im Detail gezeigt, wobei zunächst nur anhand von verschiedenen Varianten (1 bis 4) der *grundsätzliche Aufbau* bevorzugter Destillationssequenzen erläutert wird. Dieser grundsätzliche Aufbau ist selbstverständlich nicht auf die Aufarbeitung von TDI beschränkt. Im Anschluss wird dann erläutert, wie das erfindungsgemäße Verfahren in den geschilderten Destillationssequenzen umgesetzt werden kann.

### Variante 1

Die Variante 1, die sich besonders dann eignet, wenn Schritt (A) in der Flüssigphase durchgeführt wird, ist grundsätzlich beschrieben in Chem System's PERP Report for TDI/MDI (Chem Systems, Process Evaluation Research Planning TDI/MDI 98/99 S8, Tarrytown, N.Y., USA: Chem Systems 1999, S. 27 bis 32). In dieser Variante enthält das flüssige Reaktionsgemisch nach erfolgter destillativer Abtrennung von Chlorwasserstoff und Phosgen noch einen, bezogen auf seine Gesamtmasse, Lösungsmittelanteil von > 50 Massen-%, bevorzugt 51 Massen-% bis 85 Massen-%, besonders bevorzugt 55 Massen-% bis 65 Massen-%. Dieses Gemisch wird einer Lösungsmittelabtrennung zugeführt, wobei zunächst in einem Vorverdampfer ein Lösungsmittel-TDI-Gemisch in eine Lösungsmittel-Destillationskolonne abdestilliert wird. In der Lösungsmittel-Destillationskolonne wird Lösungsmittel abdestilliert und dem vorderen Abschnitt des Prozesses wieder zugeführt. Der Sumpfstrom dieser Lösungsmittel-Destillation enthält, bezogen auf die Gesamtmasse des Sumpfes, neben TDI insbesondere noch bevorzugt 15 Massen-% bis 25 Massen-% Lösungsmittel, bezogen auf die Gesamtmasse dieses Sumpfstroms. Dieser Strom wird in eine so genannte Zwischenkolonne geleitet, in der weiteres Lösungsmittel abdestilliert und das von Lösungsmittel befreite Sumpfprodukt einer letzten Destillationskolonne zur Reinigung des TDI zugeführt wird. Diese wird im Unterdruck betrieben und liefert das gereinigte verkaufsfähige Isocyanat TDI als Destillatstrom. Ein Teil des TDI verbleibt im Destillationssumpfstrom dieser letzten Destillationskolonne. Die Aufgaben der Zwischenkolonne und der Destillationskolonne zur TDI-Reinigung können auch, insbesondere wie in EP 1 371 635 A1 beschrieben, in einer Trennwandkolonne zusammengefasst werden, wobei ein Kopfstrom aus Leichtsiedern und Lösungsmittel, im Bereich der Trennwand reines TDI sowie ein TDI und höhersiedende Komponenten (Destillationsrückstand) enthaltender Produktstrom als Destillationssumpfstrom erhalten werden.

### Variante 2

Im Gegensatz zu Variante 1 enthält in dieser Ausführungsform das flüssige Reaktionsgemisch nach erfolgter destillativer Abtrennung von Chlorwasserstoff und Phosgen noch einen, auf seine Gesamtmasse bezogenen, Lösungsmittelanteil von nur ≤ 50,0 Massen-%. Dieses Gemisch wird einem Vorverdampfer zugeführt, aus dem ein Lösungsmittel-TDI-Gemisch in eine Destillationskolonne abdestilliert wird. In dieser Variante wird das TDI bereits in der letztgenannten Destillationskolonne vom Lösungsmittel befreit, sodass der Sumpfstrom dieser Destillationskolonne in die TDI-Reinigungskolonne geleitet werden kann, es mithin in dieser Variante eine Kolonne weniger gibt als in Variante 1. Die TDI-Reinigungskolonne wird im Unterdruck betrieben und liefert das gereinigte verkaufsfähige Isocyanat TDI als Destillatstrom. Die Aufgaben der TDI-Reinigungskolonne und der dieser vorgeschalteten Destillationskolonne können auch, insbesondere wie in EP 1 413 571 A1 beschrieben, in einer Trennwandkolonne zusammengefasst werden, wobei ein Kopfstrom aus Leichtsiedern und Lösungsmittel, im Bereich der Trennwand reines TDI sowie ein TDI und höhersiedende Komponenten (Destillationsrückstand) enthaltender Produktstrom als Destillationssumpfstrom erhalten werden.

### Variante 3

Die Variante 3 umfasst die in den Varianten 2 und 1 beschriebenen Destillationssequenzen, jedoch ohne den jeweils erwähnten Vorverdampfer. In diesem Fall wird der Anteil an Destillationsrückstand in den beschriebenen Destillationssequenzen über die flüssigen Mengenströme bis zur jeweiligen letzten TDI-Reinigungskolonne mitgeführt. Dieses Verfahren ist prinzipiell ebenfalls bekannt (EP 1 717 223 A2).

### Variante 4

Diese Variante wird insbesondere eingesetzt, wenn Schritt (A) in der Gasphase durchgeführt wird. Da das in einer Gasphasenphosgenierung erhaltene flüssige rohe Verfahrensprodukt gelöstes Phosgen und gelösten Chlorwasserstoff allenfalls in verhältnismäßig (d. h. verglichen mit der Flüssisphasenphosgenierung) geringer Menge enthält, wird auf eine separate Abtrennung von Phosgen und Chlorwasserstoff bevorzugt verzichtet. Das flüssige Produktgemisch wird in dieser Ausführungsform daher entweder direkt einer Lösungsmittelabtrennung zugeführt, in welcher Lösungsmittel und gegebenenfalls gelöster Chlorwasserstoff sowie gegebenenfalls gelöstes Phosgen destillativ über Kopf abgetrennt werden, oder - wenn der Lösungsmittelanteil hinreichend gering ist - es wird direkt einer TDI-Reinigungskolonne zur Rein-Destillation zugeführt. Die TDI-Reinigungskolonne ist in beiden Fällen bevorzugt als Trennwandkolonne ausgestaltet. Leichtsieder (also leichter als TDI siedende Nebenprodukte, gegebenenfalls noch vorhandener Chlorwasserstoff und gegebenenfalls noch vorhandenes Phosgen, Lösungsmittel sowie gegebenenfalls Inertgase) werden der TDI-Reinigungskolonne über Kopf entnommen. Der Kopfstrom kann darüber hinaus geringe Mengen an mitgerissenem TDI enthalten. Das gereinigte TDI wird als Destillatstrom im Bereich der Trennwand abgeführt. Der anfallende Destillationssumpfstrom enthält sog. Destillationsrückstand und eine gewisse Menge TDI, die, um den Destillationssumpfstrom verarbeitbar (fließfähig) zu halten, nicht abdestilliert wird, sowie gegebenenfalls Spurenanteile von Lösungsmittel. Anstelle einer Trennwandkolonne können selbstverständlich auch zwei in Serie geschaltete Destillationskolonnen ohne Trennwand eingesetzt werden. In dieser letztgenannten Ausführungsform werden die Leichtsieder am Kopf der ersten Destillationskolonne entnommen, wobei der Sumpfstrom der ersten Destillationskolonne den Zulauf der zweiten Destillationskolonne bildet. Gereinigtes TDI wird als Destillatstrom der zweiten Destillationskolonne entnommen, während der Sumpfstrom der zweiten Destillationskolonne den Destillationsrückstand im Gemisch mit TDI enthält.

In dieser Variante 4 wird die Lösungsmittelabtrennung - sofern durchgeführt - bevorzugt bei einer Temperatur im Bereich von 160 °C bis 200 °C und bei einem Druck im Bereich von 160 mbar bis 220 mbar durchgeführt, wobei sich beide Angaben auf den Sumpf der eingesetzten Destillationskolonne beziehen.

Die TDI-Rein-Destillation wird, insbesondere bei Durchführung in einer Trennwandkolonne, bevorzugt bei einer Temperatur im Bereich von 160 °C bis 200 °C und bei einem Druck im Bereich von 50 mbar bis 100 mbar durchgeführt, wobei sich beide Angaben auf den Sumpf der eingesetzten Destillationskolonne beziehen.

In allen geschilderten Varianten fällt also in der der Rein-Destillation des TDI ein Leichtsieder-Kopfstrom an, nämlich der Kopfstrom einer Trennwandkolonne oder der Kopfstrom einer der TDI-Reinigungskolonne ohne Trennwand vorgeschalteten Destillationskolonne. Der Hauptbestandteil dieses Leichtsieder-Kopfstromes ist eingesetztes aromatisches Lösungsmittel C₆H_{6-X}Cl_{X}. Der Gesamtmassenanteil an eingesetztem aromatischen Lösungsmittel (C₆H_{6-X}Cl_{X}) und einfach zusätzlich chloriertem aromatischem Lösungsmittel (C₆H_{6-Y}Cl_{Y}) in diesem Kopfstrom, bezogen auf dessen Gesamtmasse, beträgt insbesondere 51 % bis 99 %, besonders bevorzugt 60 % bis 99 % (wobei der Rest überwiegend bis vollständig aus TDI besteht). Dies gilt selbstverständlich nicht nur für TDI, sondern für alle Isocyanate, die sich auf die beschriebene Art und Weise (Abtrennung der Leicht- und Hochsieder in zwei hintereinandergeschalteten Destillationskolonnen oder in einer Trennwandkolonne) feinreinigen lassen. Im Stand der Technik ist es üblich, diesen in der Rein-Destillation (zunächst gasförmig) anfallenden Leichtsieder-Kopfstrom zu kondensieren und das nach Abtrennung nicht kondensierbarer Anteile erhaltene Kondensat zum Teil als Rücklauf wieder in die Rein-Destillation zurückzugeben und zum Teil aus der Rein-Destillation abzuführen und anschließend an eine andere Stelle des Prozesses zurückzuführen. Enthält der Leichtsieder-Kopfstrom jedoch substanzielle Anteile an einfach zusätzlich chloriertem aromatischem Lösungsmittel C₆H_{6-Y}Cl_{Y}, fällt also das an dieser Stelle abgetrennte aromatische Lösungsmittel der Formel C₆H_{6-X}Cl_{X} in einem Gemisch mit aromatischem Lösungsmittel der Formel C₆H_{6-Y}Cl_{Y} an, so ist diese Lösungsmittel-Rückführung problematisch, wie im Rahmen der vorliegenden Erfindung überraschend gefunden wurde. Das einfach zusätzlich chlorierte Lösungsmittel C₆H_{6-Y}Cl_{Y} reichert sich nämlich aufgrund seines vergleichsweise hohen Siedepunktes (kein nennenswerter Austrag als Gasphase, etwa gemeinsam mit nicht-kondensierbaren Gasen) sukzessive im Kondensat des Leichtsieder-Kopfstromes und damit infolge der Lösungsmittel-Rückführung im Prozess an und verunreinigt auf diese Weise letztlich das zu isolierende Isocyanat. Dem kann im Rahmen der vorliegenden Erfindung auf verschiedene Weise begegnet werden:

Die erfindungsgemäßen Anforderungen an die Reinheit des isolierten Isocyanats lassen sich in diesem Fall am einfachsten erzielen, wenn das erhaltene Gemisch, im Gegensatz zum üblichen Stand der Technik, der eine Reyzklierung des Leichtsieder-Kopfstromes an eine andere Stelle des Prozesses vorsieht, (komplett) ausgeschleust und entsorgt, insbesondere verbrannt, wird. Da hierbei jedoch Wertprodukte nur thermisch und nicht stofflich verwertet werden können, ist es bevorzugt, nur einen ersten Teil des am Kopf der Trennwandkolonne oder am Kopf der ersten Destillationskolonne erhaltenen Gemisches an eine andere Stelle des Prozesses (d. h. des Verfahrens zur Herstellung eines Isocyanats) zurückzuführen und einen zweiten Teil (in Intervallen oder kontinuierlich) auszuschleusen und anschließend zu entsorgen, insbesondere zu verbrennen (siehe auch Beispiel 2).

Die Rückführung an eine andere Stelle des Prozesses kann in Schritt (A) erfolgen oder, in Ausführungsformen umfassend die weiter oben beschriebene Wäsche des in Schritt (A) erhaltenen gasförmigen Produktgemisches mit aromatischem Lösungsmittel der Formel C₆H_{6-X}Cl_{X} zur Abtrennung von Isocyanat, bevorzugt in diese Wäsche.

Anstatt den zweiten Teil des am Kopf der Trennwandkolonne oder am Kopf der ersten Destillationskolonne erhaltenen Gemisches auszuschleusen, kann dieser zweite Teil auch in einer weiteren Destillation (die im Rahmen der Terminologie der vorliegenden Erfindung als Bestandteil der Rein-Destillation gilt) gereinigt werden, wobei aromatisches Lösungsmittel der Formel C₆H_{6-X}Cl_{X} aus dem zweiten Teil des Gemisches abgetrennt und anschließend zumindest teilweise, insbesondere vollständig, an eine andere Stelle des Prozesses zurückgeführt wird. Dazu eignen sich wie zuvor beschrieben Schritt (A) und gegebenenfalls die Wäsche des in Schritt (A) erhaltenen gasförmigen Produktgemisches (siehe Beispiel 4). Im Hinblick auf die weitere Destillation sei auf die Ausführungen weiter oben im Zusammenhang mit der Lösungsmittel-Destillation verwiesen. Das dort Gesagte gilt hier entsprechend. Insbesondere wird auch hier der Sumpfstrom der weiteren Destillation ausgeschleust und verbrannt oder anderweitig eingesetzt, jedoch nicht wieder in das Verfahren zurückgeführt. Die Verbrennung ist bevorzugt.

Es ist selbstredend ebenfalls möglich, das gesamte am Kopf der Trennwandkolonne oder am Kopf der ersten Destillationskolonne erhaltene Gemisch in einer weiteren Destillation zu reinigen und dabei aromatisches Lösungsmittel der Formel C₆H_{6-X}Cl_{X} aus dem Gemisch abzutrennen und anschließend zumindest teilweise, insbesondere vollständig, an eine andere Stelle des Prozesses zurückzuführen. Dazu eignen sich ebenfalls wieder Schritt (A) und gegebenenfalls die Wäsche des in Schritt (A) erhaltenen gasförmigen Produktgemisches.

Die *Ausschleusung* eines Teils (des zweiten Teils) des am Kopf der Trennwandkolonne oder am Kopf der ersten Destillationskolonne erhaltenen Gemisches und/oder die *Reinigung* eines Teils dieses Gemisches bzw. des gesamten Gemisches in einer *weiteren Destillation* gefolgt von der *Ausschleusung des Sumpfstromes* dieser weiteren Destillation sind dabei so vorzunehmen, dass einfach zusätzlich chloriertes aromatisches Lösungsmittel C₆H_{6-Y}Cl_{Y} in hinreichendem Maße aus dem Prozess ausgeschleust wird. Die genauen Bedingungen (wie etwa das Mengenverhältnis des ersten zum zweiten Teil des Gemisches und/oder die genaue Ausgestaltung der weiteren Destillation und/oder die Häufigkeit der Ausschleusung bei Durchführung derselben in Intervallen) sind von den Rahmenbedingungen des Einzelfalls abhängig und können vom Fachmann leicht ermittelt werden, gegebenenfalls unter Durchführung von Vorversuchen. Zu den relevanten Rahmenbedingungen gehört natürlich auch, ob einfach zusätzlich chloriertes aromatisches Lösungsmittel C₆H_{6-Y}Cl_{Y} teilweise bereits an anderen Stellen (wie der Lösungsmittel-Reinigung) ausgeschleust wurde.

Durch die erfindungsgemäße Vorgehensweise gegebenenfalls fehlende Anteile an eingesetztem aromatischem Lösungsmittel C₆H_{6-X}Cl_{X} (bei der Ausschleusung des einfach zusätzlich chlorierten aromatischen Lösungsmittels werden immer auch Anteile des eingesetzten aromatischen Lösungsmittels mit ausgeschleust) werden durch frisch zugeführtes Lösungsmittel C₆H_{6-X}Cl_{X} ersetzt. Dies gilt selbstverständlich unabhängig davon, an welcher Stelle genau einfach zusätzlich chloriertes aromatisches Lösungsmittel ausgeschleust wird.

Da Eisenchloride wie insbesondere Eisen(III)-chlorid eine Chlorierung des eingesetzten aromatischen Lösungsmittels bewirken, besteht eine Möglichkeit zur Verminderung dieser unerwünschten Chlorierungs-Reaktion (und damit zur Verminderung des Gehalts an einfach zusätzlich chloriertem aromatischem Lösungsmittel im isolierten Isocyanat) darin, an allen kritischen Stellen, an denen bekanntermaßen ein korrosiver Abtrag stattfindet oder zu befürchten ist und an denen noch signifikante Lösungsmittelkonzentrationen zugegen sind, besonders korrosionsfeste Edelstähle einzusetzen. Hierzu sind insbesondere folgende Typen geeignet:
1) Niedriggekohlte austenitische Nickel-Molybdän-Chrom Legierung mit folgenden, auf die Gesamtmasse bezogenen Massenanteilen:
   - Kohlenstoff: 0,01 % bis 0,015 %,
   - Silicium: 0 % bis 0,08 %,
   - Mangan: 0 % bis 1,00 %,
   - Phosphor: 0 % bis 0,025 %,
   - Schwefel: 0 % bis 0,015 %,
   - Chrom: 14,0 % bis 18,0 %,
   - Molybdän: 14,0 % bis 17,0 %,
   - Titan: 0 % bis 0,70 %,
   - Kupfer: 0 % bis 0,50 %,
   - Cobalt: 0 % bis 2,00 %,
   - Eisen: 0 % bis 3,00 %,
   - Nickel: Rest zu 100 %.

   - Edelstahl des Typs 2.4610, auch als "Hastelloy C4" bekannt.
2) Austenitischer Spezialedelstahl mit folgenden, auf die Gesamtmasse bezogenen Massenanteilen:
   - Kohlenstoff: 0 % bis 0,02 %,
   - Schwefel: 0 % bis 0,010 %,
   - Stickstoff: 0,15 % bis 0,25 %,
   - Chrom: 20,0 % bis 21,0 %,
   - Nickel: 24,0 % bis 26,0 %,
   - Mangan: 0 % bis 1,0 %,
   - Silicium: 0 % bis 0,5 %,
   - Molybdän: 6,0 % bis 7,0 %,
   - Kupfer: 0,5 % bis 1,5 %,
   - Phosphor: 0 % bis 0,03 %,
   - Eisen: Rest zu 100 %.

   - Edelstahl des Typs 1.4529.
3) Hochlegierter kohlenstoffarmer austenitischer Edelstahl mit folgenden, auf die Gesamtmasse bezogenen Massenanteilen:
   - Kohlenstoff: 0 % bis 0,2 %,
   - Mangan: 2 %.
   - Nickel: 23 % bis 28 %,
   - Chrom: 19 % bis 23 %,
   - Schwefel: 0 % bis 0,3 %,
   - Molybdän: 4 % bis 5 %,
   - Stickstoff: 0 % bis 0,1 %,
   - Kupfer: 1 % bis 2 %,
   - Phosphor: 0 % bis 0,03 %,
   - Silicium: 0 % bis 0,7 %,
   - Eisen: Rest zu 100 %.

   - Edelstahl des Typs 1.4539.

Zur Anwendung kommen sollten diese Edelstähle insbesondere in der Aufarbeitung ab dem zur Aufnahme des rohen flüssigen Produktgemisches der Reaktion eingesetzten Rohwarebehälter (Tankbehälter), da in der eigentlichen Reaktion (d. h. im Reaktor) meist ohnehin bereits Edelstähle von besonders hoher Qualität eingesetzt werden. Besonderes Augenmerk sollte auch auf Rohrleitungen, die zur Verbindung des Rohwarebehälters mit den eingesetzten Destillationseinrichtungen und/oder zur Verbindung dieser Destillationseinrichtungen untereinander eingesetzt werden sowie auf den Sumpfbehälter der Rein-Destillationskolonne und dessen Sumpf-Verrohrung gelegt werden.

Die Erfindung wird nachstehend anhand von Beispielen noch näher erläutert.

### Beispiele:

Prozent- und ppm-Angaben sind auf die Gesamtmasse des jeweiligen Stoffstromes bezogene Massenanteile.

Die Beispiele 1 bis 4 beschreiben die Aufarbeitung (*Schritt (B)*) von in einer Gasphasenreaktion (*Schritt (A)*) erhaltenem TDI-Produktgemisch gemäß Variante 4 (keine separate Entphosgenierung, jedoch separate Abtrennung des Hauptteils des Lösungsmittels in einer Lösungsmittelkolonne vor der Rein-Destillation; Rein-Destillation in einer Trennwandkolonne). Das in der Gasphasenreaktion als Mittel zum Abkühlen des Reaktionsgemisches eingesetzte Lösungsmittel C₆H_{6-X}Cl_{X} war ortho-Dichlorbenzol (ODB). Der in der Trennwandkolonne über Kopf abdestillierte ODB-haltige Strom wurde nach Kondensation zum Teil als Rücklauf in die Trennwandkolonne zurückgegeben und zum Teil in den Prozess zurückgeführt (nämlich in die Wäsche des in Schritt (A) nach der Quenche verbleibenden gasförmigen Reaktionsproduktes).

### Beispiel 1 (Vergleich, ohne Ausschleusung eines Stroms umfassend aromatisches Lösungsmittel der Formel C₆H_{6-Y}Cl_{Y}):

Das der Trennwandkolonne als Seitenstrom entnommene gereinigte TDI wies eine Konzentration an Trichlorbenzol (TCB) von 11 ppm auf. Der über Kopf abgezogene Leichtsiederstrom enthielt 8,6 % TDI, 88,6 % ODB und 2,2 % TCB. Höher chlorierte Chlorbenzole wie Tetra-, Penta- und Hexachlorbenzol waren mit der eingesetzten gaschromatographischen Analysen-Methode nicht detektierbar.

### Beispiel 2 (erfindungsgemäß):

Um TCB abzureichern, wurden einmalig 1,9 t des Stromes enthaltend 8,6 % TDI, 88,6 % ODB und 2,2 % TCB aus dem zur Rückführung in den Prozess bestimmten Teil ausgeschleust und einer externen Verbrennung zugeführt. Dies entspricht der erfindungsgemäßen *Ausschleusung eines Stroms umfassend aromatisches Lösungsmittel der Formel C₆H_{6-Y}Cl_{Y}.* Dabei wurde der Lösungsmittelrückführungs-Kreislauf um insgesamt 42 kg TCB abgereichert.

Diese einmalige Abreicherung an TCB im Gesamtprozess führte dazu, dass die TCB-Konzentration im TDI-Seitenstrom der Trennwandkolonne innerhalb von 12 h von 11 ppm auf 6 ppm abfiel.

### Beispiel 3 (Vergleich, ohne Ausschleusung eines Stroms umfassend aromatisches Lösungsmittel der Formel C₆H_{6-Y}Cl_{Y}; Prozess-Simulation (VTPlan):

Die Zusammensetzung des Kopfstromes der TDI-Trennwandkolonne ist wie folgt:
- 92,8 % ODB,
- 5,1 % TDI,
- 2,1 % TCB.

Der Seitenstrom der TDI-Trennwandkolonne (TDI-Produkt) enthält 15 ppm TCB.

### Beispiel 4 (erfindungsgemäß; Simulation wie in Beispiel 3):

Es werden 300 kg/h des TCB-reichen Kopfstromes aus dem zur Rückführung in den Prozess bestimmten Teil abgezweigt und in eine nachgeschaltete Destillationskolonne geleitet. Diese ist eine Packungs-Kolonne mit 24 Trennstufen, die unter 70 mbar Kopfdruck betrieben wird. In dieser nachgeschalteten Destillationskolonne wird am Kopf ODB, das einen TCB-Gehalt **unter 1 ppm** aufweist, abgetrennt. Das *so gereinigte* ODB wird nun ebenfalls in den Prozess zurückgeführt. Aus dem Sumpf dieser nachgeschalteten Destillationskolonne werden 14,5 kg/h eines **TCB-reichen Stromes** mit 3,0 % TCB, 96,9 % TDI und 0,1 % ODB entnommen, ausgeschleust und einer externen Abfallverwertung zugeführt. Dies entspricht der erfindungsgemäßen *Ausschleusung eines Stroms umfassend aromatisches Lösungsmittel der Formel C₆H_{6-Y}Cl_{Y}.*

Durch die kontinuierliche Ausschleusung, Aufarbeitung und Rückführung gereinigten Lösungsmittels ODB ändert sich die Zusammensetzung des Kopfstromes der Trennwandkolonne wie folgt:
- 95,3 % ODB,
- 4,56 % TDI,
- 0,14 % TCB.

Durch diese kontinuierliche Entnahme von Trichlorbenzol aus dem Prozess sinkt die Verunreinigung von Trichlorbenzol im TDI-Produkt von vorher 15 ppm (Beispiel 3) auf unter 3 ppm.

## Patentansprüche

1. Verfahren zur Herstellung eines Isocyanats umfassend die Schritte:
(A) Umsetzen eines Amins mit einem stöchiometrischen Überschuss an Phosgen unter Einsatz eines aromatischen Lösungsmittels der Formel C₆H_{6-X}Cl_{X}, wobei X = 1 oder 2,
(a) als Verdünnungsmittel während der Umsetzung und/oder
(b) als Mittel zum Abkühlen des durch die Umsetzung des Amins mit Phosgen entstehenden Reaktionsgemisches,
wobei ein flüssiges Produktgemisch umfassend das Isocyanat und das eingesetzte aromatische Lösungsmittel sowie ein gasförmiges Produktgemisch enthaltend Phosgen und Chlorwasserstoff erhalten werden;
(B) Isolieren des Isocyanats aus dem in Schritt (A) erhaltenen flüssigen Produktgemisch, umfassend den Schritt einer Rein-Destillation, in welcher das isolierte Isocyanat als Produktstrom anfällt, wobei in der Rein-Destillation und/oder in einem der Rein-Destillation vorgelagerten Destillationsschritt mindestens ein Strom umfassend aromatisches Lösungsmittel der Formel C₆H_{6-Y}Cl_{Y}, wobei Y = X + 1, so ausgeschleust wird, dass das isolierte Isocyanat einen auf seine Gesamtmasse bezogenen Massenanteil an aromatischem Lösungsmittel der Formel C₆H_{6-Y}Cl_{Y} im Bereich von 0,0 ppm bis 9,9 ppm aufweist.

2. Verfahren gemäß Anspruch 1, bei welchem das in Schritt (A) erhaltene flüssige Produktgemisch in Schritt (B) vor der Rein-Destillation eine Lösungsmittel-Destillation zur Abtrennung von aromatischem Lösungsmittel der Formel C₆H_{6-X}Cl_{X} durchläuft.

3. Verfahren gemäß Anspruch 1, bei welchem in Schritt (B) die Rein-Destillation
in einer Trennwandkolonne durchgeführt wird, wobei in einem Seitenabzug der Trennwandkolonne der Produktstrom an Isocyanat und am Kopf der Trennwandkolonne aromatisches Lösungsmittel der Formel C₆H_{6-X}Cl_{X} erhalten werden,
oder
in zwei hintereinandergeschalteten Destillationskolonnen ohne Trennwand durchgeführt wird, wobei am Kopf der ersten Destillationskolonne aromatisches Lösungsmittel der Formel C₆H_{6-X}Cl_{X} und als Destillat der zweiten Destillationskolonne der Produktstrom an Isocyanat erhalten werden.

4. Verfahren gemäß Anspruch 2, bei welchem in Schritt (B) die Rein-Destillation
in einer Trennwandkolonne durchgeführt wird, wobei in einem Seitenabzug der Trennwandkolonne der Produktstrom an Isocyanat und am Kopf der Trennwandkolonne aromatisches Lösungsmittel der Formel C₆H_{6-X}Cl_{X} erhalten werden,
oder
in zwei hintereinandergeschalteten Destillationskolonnen ohne Trennwand durchgeführt wird, wobei am Kopf der ersten Destillationskolonne aromatisches Lösungsmittel der Formel C₆H_{6-X}Cl_{X} und als Destillat der zweiten Destillationskolonne der Produktstrom an Isocyanat erhalten werden.

5. Verfahren gemäß Anspruch 4,
bei welchem das in der Lösungsmittel-Destillation abgetrennte aromatische Lösungsmittel der Formel C₆H_{6-X}Cl_{X} in einem Gemisch mit aromatischem Lösungsmittel der Formel C₆H_{6-Y}Cl_{Y} erhalten wird, wobei ein erster Teil des in der Lösungsmittel-Destillation erhaltenen Gemisches in Schritt (A) zurückgeführt und ein zweiter Teil des in der Lösungsmittel-Destillation erhaltenen Gemisches nicht in Schritt (A) zurückgeführt, sondern ausgeschleust wird,
oder
bei welchem das in der Lösungsmittel-Destillation abgetrennte aromatische Lösungsmittel der Formel C₆H_{6-X}Cl_{X} in einem Gemisch mit aromatischem Lösungsmittel der Formel C₆H_{6-Y}Cl_{Y} erhalten wird, wobei ein erster Teil des in der Lösungsmittel-Destillation erhaltenen Gemisches in Schritt (A) zurückgeführt und ein zweiter Teil des in der Lösungsmittel-Destillation erhaltenen Gemisches in einer weiteren Destillation gereinigt wird, wobei aromatisches Lösungsmittel der Formel C₆H_{6-X}Cl_{X} aus dem zweiten Teil des in der Lösungsmittel-Destillation erhaltenen Gemisches abgetrennt und anschließend in Schritt (A) zurückgeführt wird, wobei der nach der Abtrennung des aromatischen Lösungsmittels der Formel C₆H_{6-X}Cl_{X} aus dem zweiten Teil des in der Lösungsmittel-Destillation erhaltenen Gemisches verbleibende Teil des zweiten Teils des in der Lösungsmittel-Destillation erhaltenen Gemisches ausgeschleust wird,
oder
bei welchem das in der Lösungsmittel-Destillation abgetrennte aromatische Lösungsmittel der Formel C₆H_{6-X}Cl_{X} in einem Gemisch mit aromatischem Lösungsmittel der Formel C₆H_{6-Y}Cl_{Y} erhalten wird, wobei das in der Lösungsmittel-Destillation erhaltene Gemisch in einer weiteren Destillation gereinigt wird, wobei aromatisches Lösungsmittel der Formel C₆H_{6-X}Cl_{X} aus dem in der Lösungsmittel-Destillation erhaltenen Gemisch abgetrennt und anschließend in Schritt (A) zurückgeführt wird, wobei der nach der Abtrennung des aromatischen Lösungsmittels der Formel C₆H_{6-X}Cl_{X} aus dem in der Lösungsmittel-Destillation erhaltenen Gemisch verbleibende Teil des in der Lösungsmittel-Destillation erhaltenen Gemisches ausgeschleust wird.

6. Verfahren gemäß Anspruch 2,
bei welchem das in der Lösungsmittel-Destillation abgetrennte aromatische Lösungsmittel der Formel C₆H_{6-X}Cl_{X} in einem Gemisch mit aromatischem Lösungsmittel der Formel C₆H_{6-Y}Cl_{Y} erhalten wird, wobei ein erster Teil des in der Lösungsmittel-Destillation erhaltenen Gemisches in Schritt (A) zurückgeführt und ein zweiter Teil des in der Lösungsmittel-Destillation erhaltenen Gemisches nicht in Schritt (A) zurückgeführt, sondern ausgeschleust wird,
oder
bei welchem das in der Lösungsmittel-Destillation abgetrennte aromatische Lösungsmittel der Formel C₆H_{6-X}Cl_{X} in einem Gemisch mit aromatischem Lösungsmittel der Formel C₆H_{6-Y}Cl_{Y} erhalten wird, wobei ein erster Teil des in der Lösungsmittel-Destillation erhaltenen Gemisches in Schritt (A) zurückgeführt und ein zweiter Teil des in der Lösungsmittel-Destillation erhaltenen Gemisches in einer weiteren Destillation gereinigt wird, wobei aromatisches Lösungsmittel der Formel C₆H_{6-X}Cl_{X} aus dem zweiten Teil des in der Lösungsmittel-Destillation erhaltenen Gemisches abgetrennt und anschließend in Schritt (A) zurückgeführt wird, wobei der nach der Abtrennung des aromatischen Lösungsmittels der Formel C₆H_{6-X}Cl_{X} aus dem zweiten Teil des in der Lösungsmittel-Destillation erhaltenen Gemisches verbleibende Teil des zweiten Teils des in der Lösungsmittel-Destillation erhaltenen Gemisches ausgeschleust wird,
oder
bei welchem das in der Lösungsmittel-Destillation abgetrennte aromatische Lösungsmittel der Formel C₆H_{6-X}Cl_{X} in einem Gemisch mit aromatischem Lösungsmittel der Formel C₆H_{6-Y}Cl_{Y} erhalten wird, wobei das in der Lösungsmittel-Destillation erhaltene Gemisch in einer weiteren Destillation gereinigt wird, wobei aromatisches Lösungsmittel der Formel C₆H_{6-X}Cl_{X} aus dem in der Lösungsmittel-Destillation erhaltenen Gemisch abgetrennt und anschließend in Schritt (A) zurückgeführt wird, wobei der nach der Abtrennung des aromatischen Lösungsmittels der Formel C₆H_{6-X}Cl_{X} aus dem in der Lösungsmittel-Destillation erhaltenen Gemisch verbleibende Teil des in der Lösungsmittel-Destillation erhaltenen Gemisches ausgeschleust wird.

7. Verfahren gemäß einem der Ansprüche 3 bis 5, bei welchem das am Kopf der Trennwandkolonne oder am Kopf der ersten Destillationskolonne erhaltene aromatische Lösungsmittel der Formel C₆H_{6-X}Cl_{X} in einem Gemisch mit aromatischem Lösungsmittel der Formel C₆H_{6-Y}Cl_{Y} anfällt.

8. Verfahren gemäß Anspruch 7,
bei welchem das am Kopf der Trennwandkolonne oder am Kopf der ersten Destillationskolonne erhaltene Gemisch ausgeschleust wird,
oder
bei welchem das am Kopf der Trennwandkolonne oder am Kopf der ersten Destillationskolonne erhaltene Gemisch in einer weiteren Destillation gereinigt wird, wobei aromatisches Lösungsmittel der Formel C₆H_{6-X}Cl_{X} aus dem am Kopf der Trennwandkolonne oder am Kopf der ersten Destillationskolonne erhaltenen Gemisch abgetrennt und anschließend in Schritt (A) zurückgeführt wird, wobei der nach der Abtrennung des aromatischen Lösungsmittels der Formel C₆H_{6-X}Cl_{X} aus dem am Kopf der Trennwandkolonne oder am Kopf der ersten Destillationskolonne erhaltenen Gemisch verbleibende Teil des am Kopf der Trennwandkolonne oder am Kopf der ersten Destillationskolonne erhaltenen Gemisches ausgeschleust wird,
oder
bei welchem ein erster Teil des am Kopf der Trennwandkolonne oder am Kopf der ersten Destillationskolonne erhaltenen Gemisches in Schritt (A) zurückgeführt und ein zweiter Teil des am Kopf der Trennwandkolonne oder am Kopf der ersten Destillationskolonne erhaltenen Gemisches nicht in Schritt (A) zurückgeführt, sondern ausgeschleust wird,
oder
bei welchem ein erster Teil des am Kopf der Trennwandkolonne oder am Kopf der ersten Destillationskolonne erhaltenen Gemisches in Schritt (A) zurückgeführt und ein zweiter Teil des am Kopf der Trennwandkolonne oder am Kopf der ersten Destillationskolonne erhaltenen Gemisches in einer weiteren Destillation gereinigt wird, wobei aromatisches Lösungsmittel der Formel C₆H_{6-X}Cl_{X} aus dem zweiten Teil des am Kopf der Trennwandkolonne oder am Kopf der ersten Destillationskolonne erhaltenen Gemisches abgetrennt und anschließend in Schritt (A) zurückgeführt wird, wobei der nach der Abtrennung des aromatischen Lösungsmittels der Formel C₆H_{6-X}Cl_{X} aus dem zweiten Teil des am Kopf der Trennwandkolonne oder am Kopf der ersten Destillationskolonne erhaltenen Gemisches verbleibende Teil des zweiten Teils des am Kopf der Trennwandkolonne oder am Kopf der ersten Destillationskolonne erhaltenen Gemisches ausgeschleust wird.

9. Verfahren gemäß Anspruch 7, in Schritt (B) umfassend einen Schritt der Wäsche des in Schritt (A) erhaltenen gasförmigen Produktgemisches mit aromatischem Lösungsmittel der Formel C₆H_{6-X}Cl_{X} zur Abtrennung von Isocyanat, bei welchem
ein erster Teil des am Kopf der Trennwandkolonne oder am Kopf der ersten Destillationskolonne erhaltenen Gemisches in den Schritt der Wäsche zurückgeführt und ein zweiter Teil des am Kopf der Trennwandkolonne oder am Kopf der ersten Destillationskolonne erhaltenen Gemisches nicht in den Schritt der Wäsche zurückgeführt, sondern ausgeschleust wird,
oder
bei welchem ein erster Teil des am Kopf der Trennwandkolonne oder am Kopf der ersten Destillationskolonne erhaltenen Gemisches in den Schritt der Wäsche zurückgeführt und ein zweiter Teil des am Kopf der Trennwandkolonne oder am Kopf der ersten Destillationskolonne erhaltenen Gemisches in einer weiteren Destillation gereinigt wird, wobei aromatisches Lösungsmittel der Formel C₆H_{6-X}Cl_{X} aus dem zweiten Teil des am Kopf der Trennwandkolonne oder am Kopf der ersten Destillationskolonne erhaltenen Gemisches abgetrennt und anschließend in den Schritt der Wäsche oder in Schritt (A) zurückgeführt wird, wobei der nach der Abtrennung des aromatischen Lösungsmittels der Formel C₆H_{6-X}Cl_{X} aus dem zweiten Teil des am Kopf der Trennwandkolonne oder am Kopf der ersten Destillationskolonne erhaltenen Gemisches verbleibende Teil des zweiten Teils des am Kopf der Trennwandkolonne oder am Kopf der ersten Destillationskolonne erhaltenen Gemisches ausgeschleust wird,
oder
bei welchem das am Kopf der Trennwandkolonne oder am Kopf der ersten Destillationskolonne erhaltene Gemisch in einer weiteren Destillation gereinigt wird, wobei aromatisches Lösungsmittel der Formel C₆H_{6-X}Cl_{X} aus dem am Kopf der Trennwandkolonne oder am Kopf der ersten Destillationskolonne erhaltenen Gemisch abgetrennt und anschließend in den Schritt der Wäsche oder in Schritt (A) zurückgeführt wird, wobei der nach der Abtrennung des aromatischen Lösungsmittels der Formel C₆H_{6-X}Cl_{X} aus dem am Kopf der Trennwandkolonne oder am Kopf der ersten Destillationskolonne erhaltenen Gemisch verbleibende Teil des am Kopf der Trennwandkolonne oder am Kopf der ersten Destillationskolonne erhaltenen Gemisches ausgeschleust wird.

10. Verfahren gemäß einem der vorstehenden Ansprüche, bei welchem Rohrleitungen, die zur Verbindung eines zur Aufnahme des flüssigen Produktgemisches aus Schritt (A) eingesetzten Tankbehälters mit zur Durchführung von Schritt (B) eingesetzten Destillationseinrichtungen und/oder zur Verbindung dieser Destillationseinrichtungen untereinander eingesetzt werden, aus Edelstahl des Typs 2.4610, 1.4529 oder 1.4539 gefertigt sind.

11. Verfahren nach einem der vorstehenden Ansprüche, bei welchem der Rein-Destillation ein Produktgemisch zugeführt wird, das aromatisches Lösungsmittel der Formel C₆H_{6-X}Cl_{X} in einem auf seine Gesamtmasse bezogenen Massenanteil im Bereich von 8 % bis 49 % enthält.

12. Verfahren nach einem der vorstehenden Ansprüche, bei welchem der ausgeschleuste Strom umfassend aromatisches Lösungsmittel der Formel C₆H_{6-Y}Cl_{Y} dieses in einem auf seine Gesamtmasse bezogenen Massenanteil im Bereich von 1,0 % bis 10 % enthält.

## Claims

1. Process for preparing an isocyanate comprising the steps of:
(A) reacting an amine with a stoichiometric excess of phosgene
using an aromatic solvent of formula C₆H_{6-X}Cl_{X}, wherein X = 1 or 2,
(a) as a diluent during the reaction and/or
(b) as a means for cooling the reaction mixture formed from the reaction of the amine with phosgene
to obtain a liquid product mixture comprising the isocyanate and the employed aromatic solvent and a gaseous product mixture containing phosgene and hydrogen chloride;
(B) isolating the isocyanate from the liquid product mixture obtained in step (A) comprising the step of a final distillation in which the isolated isocyanate is obtained as a product stream, wherein in the final distillation or in a distillation step upstream of the final distillation at least one stream comprising aromatic solvent of formula C₆H_{6-Y}Cl_{Y}, wherein Y = X + 1, is discharged such that the isolated isocyanate has a mass fraction, based on its total mass, of aromatic solvent of formula C₆H_{6-Y}Cl_{Y} in the range from 0.0 ppm to 9.9 ppm.

2. Process according to Claim 1, wherein the liquid product mixture obtained in step (A) is, in step (B), prior to the final distillation, passed through a solvent distillation for separating aromatic solvent of formula C₆H_{6-X}Cl_{X}.

3. Process according to Claim 1, wherein in step (B) the final distillation
is performed in a dividing wall column to obtain the product stream of isocyanate in a sidestream takeoff from the dividing wall column and aromatic solvent of formula C₆H_{6-X}Cl_{X} at the top of the dividing wall column,
or
is performed in two serially arranged non-dividing wall distillation columns to obtain aromatic solvent of formula C₆H_{6-X}Cl_{X} at the top of the first distillation column and the product stream of isocyanate as distillate from the second distillation column.

4. Process according to Claim 2, wherein, in step (B), the final distillation
is performed in a dividing wall column to obtain the product stream of isocyanate in a sidestream takeoff from the dividing wall column and aromatic solvent of formula C₆H_{6-X}Cl_{X} at the top of the dividing wall column,
or
is performed in two serially arranged non-dividing wall distillation columns to obtain aromatic solvent of formula C₆H_{6-X}Cl_{X} at the top of the first distillation column and the product stream of isocyanate as distillate from the second distillation column.

5. Process according to Claim 4,
wherein the aromatic solvent of formula C₆H_{6-X}Cl_{X} separated in the solvent distillation is obtained in a mixture with aromatic solvent of formula C₆H_{6-Y}Cl_{Y}, wherein a first portion of the mixture obtained in the solvent distillation is recycled into step (A) and a second portion of the mixture obtained in the solvent distillation is not recycled into step (A) but is discharged,
or
wherein the aromatic solvent of formula C₆H_{6-X}Cl_{X} separated in the solvent distillation is obtained in a mixture with aromatic solvent of formula C₆H_{6-Y}Cl_{Y}, wherein a first portion of the mixture obtained in the solvent distillation is recycled into step (A) and a second portion of the mixture obtained in the solvent distillation is purified in a further distillation, wherein aromatic solvent of formula C₆H_{6-X}Cl_{X} is separated from the second portion of the mixture obtained in the solvent distillation and subsequently recycled into step (A), wherein the portion of the second portion of the mixture obtained in the solvent distillation remaining after the separation of the aromatic solvent of formula C₆H_{6-X}Cl_{X} from the second portion of the mixture obtained in the solvent distillation is discharged, or
wherein the aromatic solvent of formula C₆H_{6-X}Cl_{X} separated in the solvent distillation is obtained in a mixture with aromatic solvent of formula C₆H_{6-Y}Cl_{Y}, wherein the mixture obtained in the solvent distillation is purified in a further distillation, wherein aromatic solvent of formula C₆H_{6-X}Cl_{X} is separated from the mixture obtained in the solvent distillation and subsequently recycled into step (A), wherein the portion of the mixture obtained in the solvent distillation remaining after the separation of the aromatic solvent of formula C₆H_{6-X}Cl_{X} from the mixture obtained in the solvent distillation is discharged.

6. Process according to Claim 2,
wherein the aromatic solvent of formula C₆H_{6-X}Cl_{X} separated in the solvent distillation is obtained in a mixture with aromatic solvent of formula C₆H_{6-Y}Cl_{Y}, wherein a first portion of the mixture obtained in the solvent distillation is recycled into step (A) and a second portion of the mixture obtained in the solvent distillation is not recycled into step (A) but is discharged,
or
wherein the aromatic solvent of formula C₆H_{6-X}Cl_{X} separated in the solvent distillation is obtained in a mixture with aromatic solvent of formula C₆H_{6-Y}Cl_{Y}, wherein a first portion of the mixture obtained in the solvent distillation is recycled into step (A) and a second portion of the mixture obtained in the solvent distillation is purified in a further distillation, wherein aromatic solvent of formula C₆H_{6-X}Cl_{X} is separated from the second portion of the mixture obtained in the solvent distillation and subsequently recycled into step (A), wherein the portion of the second portion of the mixture obtained in the solvent distillation remaining after the separation of the aromatic solvent of formula C₆H_{6-X}Cl_{X} from the second portion of the mixture obtained in the solvent distillation is discharged, or
wherein the aromatic solvent of formula C₆H_{6-X}Cl_{X} separated in the solvent distillation is obtained in a mixture with aromatic solvent of formula C₆H_{6-Y}Cl_{Y}, wherein the mixture obtained in the solvent distillation is purified in a further distillation, wherein aromatic solvent of formula C₆H_{6-X}Cl_{X} is separated from the mixture obtained in the solvent distillation and subsequently recycled into step (A), wherein the portion of the mixture obtained in the solvent distillation remaining after the separation of the aromatic solvent of formula C₆H_{6-X}Cl_{X} from the mixture obtained in the solvent distillation is discharged.

7. Process according to any of Claims 3 to 5, wherein the aromatic solvent of formula C₆H_{6-X}Cl_{X} obtained at the top of the dividing wall column or at the top of the first distillation column is obtained in a mixture with aromatic solvent of formula C₆H_{6-Y}Cl_{Y}.

8. Process according to Claim 7,
wherein the mixture obtained at the top of the dividing wall column or at the top of the first distillation column is discharged,
or
wherein the mixture obtained at the top of the dividing wall column or at the top of the first distillation column is purified in a further distillation, wherein aromatic solvent of formula C₆H_{6-X}Cl_{X} is separated from the mixture obtained at the top of the dividing wall column or at the top of the first distillation column and then recycled into step (A), wherein the portion of the mixture obtained at the top of the dividing wall column or at the top of the first distillation column remaining after the separation of the aromatic solvent of formula C₆H_{6-X}Cl_{X} from the mixture obtained at the top of the dividing wall column or at the top of the first distillation column is discharged,
or
wherein a first portion of the mixture obtained at the top of the dividing wall column or at the top of the first distillation column is recycled into step (A) and a second portion of the mixture obtained at the top of the dividing wall column or at the top of the first distillation column is not recycled into step (A) but is discharged,
or
wherein a first portion of the mixture obtained at the top of the dividing wall column or at the top of the first distillation column is recycled into step (A) and a second portion of the mixture obtained at the top of the dividing wall column or at the top of the first distillation column is purified in a further distillation, wherein aromatic solvent of formula C₆H_{6-X}Cl_{X} is separated from the second portion of the mixture obtained at the top of the dividing wall column or at the top of the first distillation column and then recycled into step (A), wherein the portion of the second portion of the mixture obtained at the top of the dividing wall column or at the top of the first distillation column remaining after the separation of the aromatic solvent of formula C₆H_{6-X}Cl_{X} from the second portion of the mixture obtained at the top of the dividing wall column or at the top of the first distillation column is discharged.

9. Process according to Claim 7, comprising in step (B) a step of scrubbing the gaseous product mixture obtained in step (A) with aromatic solvent of formula C₆H_{6-X}Cl_{X} for separating isocyanate, wherein a first portion of the mixture obtained at the top of the dividing wall column or at the top of the first distillation column is recycled into the scrubbing step and a second portion of the mixture obtained at the top of the dividing wall column or at the top of the first distillation column is not recycled into the scrubbing step but is discharged, or
wherein a first portion of the mixture obtained at the top of the dividing wall column or at the top of the first distillation column is recycled into the scrubbing step and a second portion of the mixture obtained at the top of the dividing wall column or at the top of the first distillation column is purified in a further distillation, wherein aromatic solvent of formula C₆H_{6-X}Cl_{X} is separated from the second portion of the mixture obtained at the top of the dividing wall column or at the top of the first distillation column and then recycled into the scrubbing step or into step (A), wherein the portion of the second portion of the mixture obtained at the top of the dividing wall column or at the top of the first distillation column remaining after the separation of the aromatic solvent of formula C₆H_{6-X}Cl_{X} from the second portion of the mixture obtained at the top of the dividing wall column or at the top of the first distillation column is discharged,
or
wherein the mixture obtained at the top of the dividing wall column or at the top of the first distillation column is purified in a further distillation, wherein aromatic solvent of formula C₆H_{6-X}Cl_{X} is separated from the mixture obtained at the top of the dividing wall column or at the top of the first distillation column and then recycled into the scrubbing step or into step (A), wherein the portion of the mixture obtained at the top of the dividing wall column or at the top of the first distillation column remaining after the separation of the aromatic solvent of formula C₆H_{6-X}Cl_{X} from the mixture obtained at the top of the dividing wall column or at the top of the first distillation column is discharged.

10. Process according to any of the preceding claims, wherein pipe conduits which are used for connecting a tank container for receiving the liquid product mixture from step (A) with distillation means for performing step (B) and/or for connecting these distillation means to one another are fabricated from stainless steel of type 2.4610, 1.4529 or 1.4539.

11. Process according to any of the preceding claims, wherein the final distillation is supplied with a product mixture comprising aromatic solvent of formula C₆H_{6-X}Cl_{X} in a mass fraction based on its total mass in the range from 8% to 49%.

12. Process according to any of the preceding claims, wherein the discharged stream comprising aromatic solvent of formula C₆H_{6-Y}Cl_{Y} contains said solvent in a mass fraction based on its total mass in the range from 1.0% to 10%.

## Revendications

1. Procédé de préparation d'un isocyanate, comprenant les étapes :
(A) réaction d'une amine avec un excès stoechiométrique de phosgène avec utilisation d'un solvant aromatique de formule C₆H_{6-X}Cl_{X}, avec X = 1 ou 2,
(a) en tant que diluant pendant la réaction et/ou
(b) en tant que moyen de refroidissement du mélange réactionnel obtenu par la réaction de l'amine avec du phosgène,
avec obtention d'un mélange de produits liquides comprenant l'isocyanate et le solvant aromatique utilisé, ainsi qu'un mélange de produits gazeux contenant du phosgène et du chlorure d'hydrogène ;
(B) isolement de l'isocyanate du mélange de produits liquides obtenu dans l'étape (A), comprenant l'étape d'une distillation pure, dans laquelle l'isocyanate isolé est obtenu sous forme de courant de produits ; dans la distillation pure et/ou dans une étape de distillation en amont de la distillation pure, au moins un courant comprenant un solvant aromatique de formule C₆H_{6-Y}Cl_{Y}, avec Y = X + 1, étant évacué de telle sorte que l'isocyanate isolé présente une proportion en masse du solvant aromatique de formule C₆H_{6-Y}Cl_{Y}, rapportée à sa masse totale, dans la plage de 0,0 ppm à 9,9 ppm.

2. Procédé selon la revendication 1, dans lequel le mélange de produits liquides obtenu dans l'étape (A) passe dans l'étape (B) avant la distillation pure par une distillation du solvant, destiné à séparer le solvant aromatique de formule C₆H_{6-X}Cl_{X}.

3. Procédé selon la revendication 1, dans lequel dans l'étape (B) la distillation pure
est mise en oeuvre dans une colonne à cloison, avec obtention du courant de produits en isocyanate dans un soutirage latéral de la colonne à cloison et, en tête de la colonne à cloison, d'un solvant aromatique de formule C₆H_{6-X}Cl_{X},
ou
est mise en oeuvre dans deux colonnes de distillation successives sans cloison, avec obtention en tête de la première colonne de distillation d'un solvant aromatique de formule C₆H_{6-X}Cl_{X} et, en tant que distillat de la deuxième colonne de distillation, le courant de produits en isocyanate.

4. Procédé selon la revendication 2, dans lequel dans l'étape (B) la distillation pure
est mise en oeuvre dans une colonne à cloison, avec obtention dans un soutirage latéral de la colonne à cloison du courant de produits en isocyanate et, en tête de la colonne à cloison, d'un solvant aromatique de formule C₆H_{6-X}Cl_{X},
ou
est mise en oeuvre dans deux colonnes de distillation successives sans cloison, avec obtention en tête de la première colonne de distillation d'un solvant aromatique de formule C₆H_{6-X}Cl_{X} et, en tant que distillat de la deuxième colonne de distillation, le courant de produits en isocyanate.

5. Procédé selon la revendication 4,
dans lequel le solvant aromatique de formule C₆H_{6-X}Cl_{X} séparé dans la distillation des solvants est obtenu dans un mélange avec un solvant aromatique de formule C₆H_{6-Y}Cl_{Y}, une première partie du mélange obtenu dans la distillation des solvants étant renvoyée dans l'étape (A) et une deuxième partie du mélange obtenu dans la distillation des solvants n'étant pas renvoyée dans l'étape (A), mais évacuée, ou
dans lequel le solvant aromatique de formule C₆H_{6-X}Cl_{X} séparé dans la distillation des solvants est obtenu dans un mélange avec un solvant aromatique de formule C₆H_{6-Y}Cl_{Y}, une première partie du mélange obtenu dans la distillation des solvants étant renvoyée dans l'étape (A) et une deuxième partie du mélange obtenu dans la distillation des solvants étant purifiée dans une autre distillation, le solvant aromatique de formule C₆H_{6-X}Cl_{X} étant séparé de la deuxième partie du mélange obtenu dans la distillation des solvants, puis étant renvoyé dans l'étape (A), la partie de la deuxième partie du mélange obtenu dans la distillation des solvants, restant après séparation du solvant aromatique de formule C₆H_{6-X}Cl_{X} de la deuxième partie du mélange obtenu dans la distillation des solvants, étant évacuée,
ou
dans lequel le solvant aromatique de formule C₆H_{6-X}Cl_{X} séparé dans la distillation des solvants est obtenu dans un mélange avec un solvant aromatique de formule C₆H_{6-Y}Cl_{Y}, le mélange obtenu dans la distillation des solvants étant purifié dans une autre distillation, le solvant aromatique de formule C₆H_{6-X}Cl_{X} étant séparé du mélange obtenu dans la distillation des solvants puis renvoyé dans l'étape (A), la partie du mélange obtenu dans la distillation des solvants restant après la séparation du solvant aromatique de formule C₆H_{6-X}Cl_{X} du mélange obtenu dans la distillation des solvants, étant évacuée.

6. Procédé selon la revendication 2,
dans lequel le solvant aromatique de formule C₆H_{6-X}Cl_{X} séparé dans la distillation des solvants est obtenu dans un mélange avec un solvant aromatique de formule C₆H_{6-Y}Cl_{Y}, une première partie du mélange obtenu dans la distillation des solvants étant renvoyée dans l'étape (A) et une deuxième partie du mélange obtenu dans la distillation des solvants n'étant pas renvoyée dans l'étape (A), mais évacuée,
ou
dans lequel le solvant aromatique de formule C₆H_{6-X}Cl_{X} séparé dans la distillation des solvants est obtenu dans un mélange avec un solvant aromatique de formule C₆H_{6-Y}Cl_{Y}, une première partie du mélange obtenu dans la distillation des solvants étant renvoyée dans l'étape (A) et une deuxième partie du mélange obtenu dans la distillation des solvants étant purifiée dans une autre distillation, le solvant aromatique de formule C₆H_{6-X}Cl_{X} étant séparé de la deuxième partie du solvant obtenu dans la distillation des solvants, puis étant renvoyé dans l'étape (A), la deuxième partie du mélange obtenu dans la distillation des solvants, restant après la séparation du solvant aromatique de formule C₆H_{6-X}Cl_{X} de la deuxième partie du mélange obtenu dans la distillation des solvants, étant évacuée,
ou
dans lequel le solvant aromatique de formule C₆H_{6-X}Cl_{X} séparé dans la distillation des solvants est obtenu dans un mélange avec un solvant aromatique de formule C₆H_{6-Y}Cl_{Y}, le mélange obtenu dans la distillation des solvants étant purifié dans une autre distillation, le solvant aromatique de formule C₆H_{6-X}Cl_{X} étant séparé du mélange obtenu dans la distillation des solvants puis renvoyé dans l'étape (A), la partie du mélange obtenu dans la distillation des solvants, restant après la séparation du solvant aromatique de formule C₆H_{6-X}Cl_{X} du mélange obtenu dans la distillation des solvants, étant évacuée.

7. Procédé selon l'une des revendications 3 à 5, dans lequel le solvant aromatique de formule C₆H_{6-X}Cl_{X} obtenu en tête de la colonne à cloison ou en tête de la première colonne de distillation arrive dans un mélange avec un solvant aromatique de formule C₆H_{6-Y}Cl_{Y}.

8. Procédé selon la revendication 7,
dans lequel le mélange obtenu en tête de la colonne à cloison ou en tête de la première colonne de distillation est évacué,
ou
dans lequel le mélange obtenu en tête de la colonne à cloison ou en tête de la première colonne de distillation est purifié dans une autre distillation, le solvant aromatique de formule C₆H_{6-X}Cl_{X} étant séparé du mélange obtenu en tête de la colonne à cloison ou en tête de la première colonne de distillation et étant ensuite renvoyé dans l'étape (A), la partie du mélange obtenu en tête de la colonne à cloison ou en tête de la première colonne de distillation, restant après la séparation du solvant aromatique de formule C₆H_{6-X}Cl_{X} du mélange obtenu en tête de la colonne à cloison ou en tête de la première colonne de distillation, étant évacuée,
ou
dans lequel une première partie du mélange obtenu en tête de la colonne à cloison ou en tête de la première colonne de distillation étant renvoyée dans l'étape (A), et une deuxième partie du mélange obtenu en tête de la colonne à cloison ou en tête de la première colonne de séparation n'étant pas renvoyée dans l'étape (A), mais évacuée,
ou
dans lequel une première partie du mélange obtenu en tête de la colonne à cloison ou en tête de la première colonne de distillation est renvoyée dans l'étape (A) et une deuxième partie du mélange obtenu en tête de la colonne à cloison ou en tête de la première colonne de distillation est purifiée dans une autre distillation, le solvant aromatique de formule C₆H_{6-X}Cl_{X} étant séparé de la deuxième partie du mélange obtenu en tête de la colonne à cloison ou en tête de la première colonne de distillation et étant ensuite renvoyé dans l'étape (A), la partie de la deuxième partie du mélange obtenu en tête de la colonne à cloison ou en tête de la première colonne de distillation, restant après la séparation du solvant aromatique de formule C₆H_{6-X}Cl_{X} de la deuxième partie du mélange obtenu en tête de la colonne à cloison ou en tête de la première colonne de distillation, étant évacuée.

9. Procédé selon la revendication 7, comprenant dans l'étape (B) une étape de lavage du mélange de produits gazeux obtenu dans l'étape (A) avec un solvant aromatique de formule C₆H_{6-X}Cl_{X} pour séparer l'isocyanate, dans lequel
une première étape du mélange obtenu en tête de la colonne à cloison ou en tête de la première colonne de distillation est renvoyée dans l'étape de lavage et une deuxième étape du mélange obtenu en tête de la colonne à cloison ou en tête de la première colonne de distillation n'est pas renvoyée dans l'étape de lavage, mais est évacuée,
ou
dans lequel une première partie du mélange obtenu en tête de la colonne à cloison ou en tête de la première colonne de distillation est renvoyée dans l'étape de lavage et une deuxième partie du mélange obtenu en tête de la colonne à cloison ou en tête de la première colonne de distillation est purifiée dans une autre distillation, le solvant aromatique de formule C₆H_{6-X}Cl_{X} étant séparé de la deuxième partie du mélange obtenu en tête de la colonne à cloison ou en tête de la première colonne de distillation puis étant renvoyé dans l'étape de lavage ou dans l'étape (A), la partie de la deuxième partie du mélange obtenu en tête de la colonne à cloison ou en tête de la première colonne de distillation, restant après la séparation du solvant aromatique de formule C₆H_{6-X}Cl_{X} de la deuxième partie du mélange obtenu en tête de la colonne à cloison ou en tête de la première colonne de distillation, étant évacuée,
ou
dans lequel le mélange obtenu en tête de la colonne à cloison ou en tête de la première colonne de distillation est purifié dans une autre distillation, le solvant aromatique de formule C₆H_{6-X}Cl_{X} étant séparé du mélange obtenu en tête de la colonne à cloison ou en tête de la première colonne de distillation et étant ensuite renvoyé dans l'étape de lavage ou dans l'étape (A), la partie du mélange obtenu en tête de la colonne à cloison ou en tête de la première colonne de distillation, restant après la séparation du solvant aromatique de formule C₆H_{6-X}Cl_{X} du mélange obtenu en tête de la colonne à cloison ou en tête de la première colonne de distillation, étant évacuée.

10. Procédé selon l'une des revendications précédentes, dans lequel les tuyauteries qui sont utilisées pour relier un réservoir utilisé pour la réception du mélange de produits liquides de l'étape (A) à des dispositifs de distillation utilisés pour la mise en oeuvre de l'étape (B) et/ou pour relier les uns aux autres ces dispositifs de distillation, sont fabriquées en acier spécial de type 2.4610, 1.4529 ou 1.4539.

11. Procédé selon l'une des revendications précédentes, dans lequel on envoie à la distillation pure un mélange de produits qui contient un solvant aromatique de formule C₆H_{6-X}Cl_{X} selon une proportion en masse, rapportée à sa masse totale, dans la plage de 8 % à 49 %.

12. Procédé selon l'une des revendications précédentes, dans lequel le courant évacué comprenant un solvant aromatique de formule C₆H_{6-Y}Cl_{Y} contient ce dernier selon une proportion en masse, rapportée à sa masse totale, dans la plage de 1,0 % à 10 %.
